(19) Europäisches Patentamt
European Patent Office
Office européen des brevets

(11) Publication number : **0 624 368 A2**

(12) **EUROPEAN PATENT APPLICATION**

(21) Application number : **94303169.0**

(22) Date of filing : **29.04.94**

(51) Int. Cl.$^5$ : **A61K 31/425,** A61K 31/505, A61K 31/195

(30) Priority : **10.05.93 US 59775**

(43) Date of publication of application : **17.11.94 Bulletin 94/46**

(84) Designated Contracting States : **BE CH DE ES FR GB IT LI SE**

(71) Applicant : **FREE RADICAL SCIENCES, Inc.**
**245 First Street,**
**14th Floor**
**Cambridge, Massachusetts 02142 (US)**

(72) Inventor : **Goldberg, Dennis I.**
**3 Newhaven Drive**
**Hawthorn Woods, Illinois 60047 (US)**
Inventor : **Sun, Chong-Son**
**530 Golf Lane**
**Lake Forest, Illinois 60045 (US)**
Inventor : **Josephs, Steven**
**366 Clarewood Circle**
**Grayslake, Illinois 60030 (US)**

(74) Representative : **MacGregor, Gordon**
**ERIC POTTER & CLARKSON**
**St. Mary's Court**
**St. Mary's Gate**
**Nottingham, NG1 1LE (GB)**

(54) **Use of a composition for stimulating the synthesis of glutathione in the manufacture of a medicament for the treatment of acquired immune deficiency syndrome.**

(57) The present invention provides a method for treating AIDS. The method comprises the step of administering a therapeutically effective amount of an antiretroviral agent and a therapeutically effective amount of an intracellular glutathione stimulator to an HIV positive patient. In a preferred embodiment, the antiretroviral agent comprises AZT. In an embodiment, the composition for stimulating the intracellular glutathione levels comprises L-2-oxothiazolidine-4-carboxylate.

EP 0 624 368 A2

## BACKGROUND OF THE INVENTION

The present invention relates generally to the treatment of disease states in patients. More specifically, the present invention relates to the treatment of acquired immunodeficiency syndrome (AIDS)

Presently, 3'-Azidothymidine (AZT) is the primary therapy for the treatment of AIDS. AZT is an antiretroviral drug that is active against human immunodeficiency virus (HIV). In this regard, AZT is an inhibitor of the in vitro replication of some retroviruses, including HIV. AZT is marketed by the Burroughs Wellcome Company under the name Retrovir. Retrovir is the brand name for Zidovudine (formerly called azidothymidine (AZT)). See Physician Desk Reference, 44th Edition, 1990, pg. 799.

Recently, attention has been focussed on another potential treatment for AIDS using DDI. DDI is also an antiretroviral drug.

Unfortunately, AZT has a significant dose dependent toxicity. In fact, the 1990 PDR bears the following statement:

Warning

Therapy with Retrovoir (Zidovudine) is often associated with hematologic toxicity including granulocytopenia and severe anemia requiring transfusions (see warnings).

AZT related bone marrow toxicity limits the utility of the drug. See, Thompson et al, "Hematologic Toxicity of AZT and ddC Administered as Single Agent and in Combination to Rats and Mice", Fundamental and Applied Toxicity, 17, 159-176 (1991). Although the basis of the toxicity is not completely understood, some evidence suggests a metabolite of AZT, 3'-amino-3'-deoxythymidine (AMT), may be at least partially responsible. See, Cretton, "Catabolism of 3'-Azido-3'-deoxythymidine in Hepatocytes in Liver Microsomes, with Evidence of Formation of 3'-Amino-3'-deoxythymidine, a Highly Toxic Catabolite for Human Bone Marrow Cells", Molecular Pharmacology, 39:258-266. It has been reported that some thiols, including reduced glutathione, can reduce AZT to AMT in vivo. See, Handlon et al, "Thiol Reduction of 3'-Azidothymidine to 3'-Aminothymidine: Kinetics and Biomedical Implications", Pharmaceutical Research, Vol. 5, No. 5, 1988.

Additionally, long term administration of AZT can cause mitochondrial mycotoxicity. See, Lamperth et al, "Abnormal Skeletal and Cardiac Muscle Mitochondria Induced by Zidovudine (AZT) in Human Muscles In Vitro and in Animal Model", Laboratory Investigation, 1991, 65:742. This results in a myopathy that develops after a mean period of 12 months of therapy. Id.

Further adverse reactions have been reported with AZT. Additionally, DDI has been reported to also cause a variety of severe adverse reactions.

Due to the dose dependent toxicity of AZT and DDI the effectiveness of these drugs as therapies in treating AIDS has been limited. further, although currently these therapies are the only recognized therapies for the treatment of AIDS, their use is not entirely satisfactory. There is therefore a need for an improved AIDS therapy and method of treatment.

## SUMMARY OF THE INVENTION

The present invention provides a method of treating acquired immune deficiency syndrome (AIDS). By administering a glutathione stimulator with an antiretroviral agent, the effectiveness of the antiretroviral agent can be potentiated. This results in a more efficacious treatment of AIDS than heretofore possible.

To this end, in an embodiment, the present invention provides a method of treating AIDS. Pursuant to the present invention, a method of treating AIDS is provided comprising administering to a patient a therapeutically effective amount of an antiretroviral agent and a therapeutically effective amount of a composition that stimulates the intracellular synthesis of glutathione.

The composition can be administered either parenterally or enterally.

In a preferred embodiment, the composition includes L-2-oxothiazolidine-4-carboxylate and is used with AZT.

However, other compositions that stimulate the intracellular synthesis of glutathione can be utilized. These compositions include esters of L-2-oxothiazolidine-4-carboxylate; glutathione esters; thiazolidine-4-carboxylate analogs; and proteins rich in cysteine. Such proteins can include: whey; egg whites; serum albumin; and lactalbumin.

Additionally, the present invention provides a method for potentiating the antiviral effect of an antiretroviral agent comprising the step of administering a therapeutically effective amount of an antiretroviral compound and administering a therapeutically effective amount of a composition that stimulates the intracellular synthesis of glutathione.

An advantage of the present invention is to provide an improved method for treating AIDS.

Still further, an advantage of the present invention is to provide a method for potentiating the effectiveness

of an antiretroviral compound.

Furthermore, an advantage of the present invention is to provide compositions for more effectively treating AIDS.

Moreover, an advantage of the present invention is to provide a composition that can be used to treat AIDS and does not have the severe side effects of presently utilized compositions.

Additional features and advantages of the present invention are described in, and will be apparent from, the detailed description of the presently preferred embodiments.

## DETAILED DESCRIPTION OF THE PRESENTLY PREFERRED EMBODIMENTS

The treatment of HIV and/or AIDS infection is currently not satisfactory. Although it is known to use antiretroviral therapy, such therapy is believed only to delay the ultimate fatality of AIDS. In part, the use of antiretroviral therapy is limited due to the severe toxicity associated with such agents. For example, AZT causes a dose dependent hemotoxicity. DDI also induces severe life threatening adverse side effects. The toxicity of such antiretroviral agents is dose dependent thereby limiting the therapeutic approaches that can be taken with such compounds.

The inventors have surprisingly found that by providing a treatment including an intracellular glutathione stimulator in combination with AZT, a more efficacious treatment for AIDS is provided. In this regard, the administration of, for example, L-2-oxothiazolidine-4-carboxylate, in combination with AZT, results in a copotentiation of the antiviral efficacy of AZT. This is demonstrated in Example No. 2 set forth below.

Thus, the present invention provides an improved treatment for AIDS that will prolong the life expectancy of an AIDS patient. The method comprises the steps of administering a glutathione stimulator along with AZT to a patient suffering from AIDS or who is HIV positive.

The inventors have also surprisingly found that an intracellular glutathione stimulator when administered to a patient taking antiretroviral compounds will reduce or prevent the toxicity associated with the antiretroviral therapy. This is a surprising result in view of the fact that it had been reported that reduced glutathione, and other thiols, may reduce AZT to AMT. See, Handlon et al, <u>supra</u>.

This belief, coupled with the evidence suggesting that AMT may be at least partially responsible for the bone marrow toxicity of AZT (see, Cretton, <u>supra</u>) would lead one away from such a combination. Surprisingly, the inventors have found that an intracellular glutathione stimulator will reduce the toxicity of AZT as well as potentiate the effectiveness of AZT as an antiretroviral agent. As set forth in detail below in the experiment described in Example No. 1, the inventors have specifically found that L-2-oxothiazolidine-4-carboxylate will reduce the toxicity of AZT.

L-2-oxothiazolidine-4-carboxylate, <u>in vitro</u>, is subjected to the action of 5-oxo-L-prolinase in the presence of adenosine triphosphate to produce S-carboxyl cysteine. S-carboxyl cysteine is then decarboxylated to produce cysteine. Cysteine is then metabolized to provide glutathione. See, U.S. Patent Nos.: 4,335,210; 4,434,158; 4,438,124; 4,665,082; and 4,647,571, the disclosures of which are incorporated herein by reference.

Likewise, esters of L-2-oxothiazolidine-4-carboxylate can be used. The ester can be saturated straight or branched, alkyl group of 1 to 10 carbon atoms. Preferably, the ester is chosen from a saturated straight alkyl group such as methyl, ethyl, propyl, butyl, pentyl, hexyl, heptyl, octyl, nonyl, or decyl and a saturated branched alkyl group such as isopropyl, isobutyl, sec-butyl, tert-butyl, or isopenyl. Such esters are disclosed in U.S. Patent 5,208,249, the disclosure of which is hereby incorporated herein by reference.

The inventors also believe that other substrates that stimulate intracellular glutathione synthesis can also be utilized in combination with antiretroviral drugs, such as AZT, to treat AIDS pursuant to the present invention. To this end, the inventors believe that glutathione esters, as well as other thiazolidine-4-carboxylate analogs, that are converted intracellularly the glutathione can be utilized.

Such a glutathione ester, for example, can have the structure:

$$HOOC{-}{-}\underset{NH_2}{\underset{|}{CH}}CH_2CH_2\ CONH\underset{CH_2SH}{\underset{|}{CH}}CONHCH_2COOR$$

wherein: R is an alkyl group containing 1 to 10 carbon atoms. Preferably, the methyl and ethyl glutathione esters are used. Glutathione esters are disclosed in U.S. Patent No. 4,710,489, the disclosure of which is incorporated herein by reference.

Further, the inventors postulate that proteins enriched in cysteine, that will also stimulate intracellular glutathione synthesis, can also be utilized. Such proteins, if they have sufficiently high cysteine levels and are administered in sufficiently high quantities, it is believed, can provide the necessary intracellular glutathione

stimulation. For example, the following proteins have a high cysteine content: whey (2%); egg white (2.5%); serum albumin (5.5%); and lactalbumin (5.8%).

Additionally, other intracellular glutathione stimulators, such as N-acetyl cysteine, may be used.

Pursuant to the present invention, the compositions of the present invention can either be administered parenterally or enterally. Enterally, the composition can comprise a pill including, for example, L-2-oxothiazolidine-4-carboxylate that is administered to a patient either contemporaneously with the antiretroviral drug or on the same days. Likewise, the composition can include an enteral solution that includes the intracellular glutathione stimulator. It is even envisioned that the composition can be part of the patients' diet.

Parenterally, the composition can, for example, include approximately 3% to about 6%, by weight, L-2-oxothiazolidine-4-carboxylate in a buffer solution such as a phosphate buffer. Of course, parenterally, the composition can be administered as a bolus or as an additive to an IV solution.

By way of example, and not limitation, examples of the present invention will now be given.

## EXAMPLE NO. 1

To evaluate the hematologic response (peripheral blood and bone marrow), mice were treated by gavage twice daily for 14 days with 3'-azido-3'-deoxythymidine (AZT) while being administered L-2-oxothiazolidine-4-carboxylate (Procysteine®) orally with the feed.

L-2-oxothiazolidine-4-carboxylate (Procysteine®), was provided by Clintec Nutrition Company, Deerfield, Illinois. Diet (MEAL) containing 1.0% (wt/wt) Procysteine® was formulated. The control diet consisted of MEAL containing no Procysteine®.

3'-azido-3'-deoxythymidine (AZT) was provided by Sigma Chemical Company, St. Louis, Missouri. AZT was formulated as a suspension at 2.5 and 25 mg/mL in sterile water containing 0.5% (wt/vol) methylcellulose. AZT suspensions were dispensed into amber glass bottles. Enough AZT to treat animals in all groups/blocks for the duration of the study was made one day prior to study day 3 for block 1.

0.5% (wt/vol) methylcellulose in sterile water was used as the vehicle control. The vehicle control suspension was dispensed and handled in a similar fashion as the positive control article. Enough of the vehicle control to dose animals in all groups/blocks for the duration of the study was made one day prior to study day 3 for block 1.

The control diet and diet containing Procysteine® were stored at room temperature in sealed containers in the study room. While in a fume hood, the diet containing Procysteine® was weighed and dispensed into stainless steel feeders. In a fume hood, AZT was weighed, formulated into a suspension and dispensed into bottles.

The vehicle control suspension and AZT suspensions were stored refrigerated at 5±3°C. At each gavage dosing period, one bottle of each of the suspensions was removed from the refrigerator approximately 30-60 minutes before dosing and maintained with continual stirring using a magnetic stir bar until the completion of dosing. While in a fume hood, mice were treated by gavage twice a day, starting on study day 3.

Sixty male and sixty female mice from Charles River Laboratories, Portage, Michigan, were studied. Mice were approximately 7-8 weeks old at the start of AZT dosing. On study day 0, the weight of the mice was as follows: males: 17-30 g; and females: 14-25 g. Only mice showing no signs of clinical illness were used in this study. The mice were from a colony which was raised and certified by the vendor to be free of pathogens as indicated upon receipt.

The experimental design for treating the animals was as shown in Table 1. Three days prior to the initiation of AZT treatment (i.e., on study days 0, 1, and 2), mice in groups 2, 5, and 6 were placed on the diet containing 1.0% (wt/wt) Procysteine®. Mice in groups 1, 3, and 4 were placed on the control diet starting on study day 0.

Treatment with these diets was continued on study days 3-16.

Starting on study day 3, mice in groups 1 and 2 were treated by oral gavage with a vehicle control (0.5% methyl-cellulose in distilled water) or, in groups 3, 4, 5, and 6, with a positive control article (AZT at 50 or 500 mg/kg body weight/day), as appropriate. Mice were weighed before the first treatment every day to calculate dosage. Mice were treated by gavage twice daily, starting at approximately 9 a.m. and 3 p.m., receiving 1/2 of the daily dose at each treatment. At each time point, mice were treated using a volume of approximately 10 mL/kg body weight.

Animals were weighed (not fasted) and sacrificed on study day 17. At this time, blood was collected for hematology evaluation, and bone marrow was collected for bone marrow and stem cell analysis.

**Table 1.**
**Scheme for Treating Mice with AZT and Procysteine®**

| Group No. | AZT Daily Dosage[1] (mg/kg) (starting day 3) | Procysteine® (% of Diet) (starting Day 0) | No. Mice/Group (male/female) |
|---|---|---|---|
| 1 | 0 | 0 | 10/10 |
| 2 | 0 | 1.0 | 10/10 |
| 3 | 50 | 0 | 10/10 |
| 4 | 500 | 0 | 10/10 |
| 5 | 50 | 1.0 | 10/10 |
| 6 | 500 | 1.0 | 10/10 |

[1]AZT was given by gavage at 1/2 the daily dosage, twice daily for 14 days in a volume of 10 mL/kg body weight. AZT was formulated at 2.5 and 25 mg/mL for the 50 and 500 mg/kg body weight treatments, respectively.

Mice received Certified Rodent Diet - MEAL (Ralston Purina Co., St. Louis, Missouri). Three days prior to the initiation of AZT, mice in groups 2, 5, and 6 were placed on diet containing 1.0% (wt/wt) Procysteine®. The diet was put into stainless steel dishes placed in each cage. Food consumption was monitored on a daily basis for all animals within a block upon the initiation of Procysteine® feeding to animals within that block.

Deionized drinking water was provided ad libitum through demand-controlled valves in each cage. The water was periodically analyzed for microbial and chemical contaminants.

To accommodate the necropsy procedure, animals were assigned to 1 of 5 blocks (24 mice/block) with initiation of treatment occurring on 5 consecutive days (i.e., study day 0 for block 2 is one day after study day 0 for block 1, and so on) (see Table 2). Male mice were numbered from 1 through 60, female mice from 61 through 120, according to their body weight on study day 0 for block 1. For a given sex of animal, the heavier the animal, the smaller the number assigned. If any mice in groups 1, 3 or 4 died immediately after their first gavage treatment, they were replaced in the study, if possible. The assignment of animals to treatment groups (5 blocks) is given in Table 2.

Mice were treated and necropsied according to block, in ascending group order; per block, males were treated first, followed by females. For example, Procysteine® treatment started for block 1 on study day 0; the next day was study day 1 for block 1 and study day 0 for block 2, and so on. At any given gavage period (a.m. or p.m.), all males and females of a given block were treated before mice from the next block are started.

**Table 2. Systematic Assignment of Animals**

| Group | Sex | 1 | 2 | 3 | 4 | 5 |
|---|---|---|---|---|---|---|
| 1 | M | 01, 52 | 13, 44 | 25, 36 | 07, 58 | 19, 50 |
| | F | 61, 112 | 73, 104 | 85, 96 | 67, 118 | 79, 110 |
| 2 | M | 12, 41 | 24, 33 | 06, 55 | 18, 47 | 30, 39 |

| | | | | | | |
|---|---|---|---|---|---|---|
| | F | 72, 101 | 84, 93 | 66, 115 | 78, 107 | 90, 99 |
| 3 | M | 21, 32 | 03, 54 | 15, 46 | 27, 38 | 09, 60 |
| | F | 81, 92 | 63, 114 | 75, 106 | 87, 98 | 69, 120 |
| 4 | M | 02, 51 | 14, 43 | 26, 35 | 08, 57 | 20, 49 |
| | F | 62, 111 | 74, 103 | 86, 95 | 68, 117 | 80, 109 |
| 5 | M | 11, 42 | 23, 34 | 05, 56 | 17, 48 | 29, 40 |
| | F | 71, 102 | 83, 94 | 65, 116 | 77, 108 | 89, 100 |
| 6 | M | 22, 31 | 04, 53 | 16, 45 | 28, 37 | 10, 50 |
| | F | 82, 91 | 64, 113 | 76, 105 | 88, 119 | |

At approximately 3:00 p.m. on study day 0, animals were placed on a measured amount of diet (at least approximately 11 g/day) containing 0 or 1% Procysteine®, as appropriate. Feed was administered in stainless steel dishes placed in each cage. At approximately 7:00 a.m. on each of the succeeding days, the feed containers were removed, weighed, cleaned, and fresh diet added; containers were returned to the cages at approximately 3:00 p.m. each day except study day 17. On study days 3-16, each animal was treated by gavage twice daily, starting at approximately 9:00 a.m. and 3:00 p.m., with approximately 10 ml/kg body weight of the vehicle control, or the positive control (AZT) at 50 or 500 mg/kg body weight.

Starting on study day 0, body weights were recorded daily.

Each animal was observed approximately 1 hour after being treated by gavage.

On study day 17, the animals were anesthetized with methoxyflurane (in a bell jar). When deeply anesthetized, blood was obtained from the abdominal aorta and transferred for hematological analysis into tubes containing an $K_2EDTA$ suspension.

Bone marrow smear slides were prepared by extracting a small quantity of bone marrow from a femur using a small brush previously dipped in mouse serum and then brushing the marrow onto the slides. The bone marrow slides were dipped (five times for approximately one second each time) into Diff-Quik® fixative and allowed to air dry. Prepared slides were evaluated microscopically.

The following assays were conducted.

Leukocyte count
Erythrocyte count
Total hemoglobin concentration
Hematocrit
Mean corpuscular volume (MCV)
Mean corpuscular hemoglobin (MCH)
Mean corpuscular hemoglobin concentration
Platelet count
Reticulocyte count
Differential leukocyte count

The samples were processed and data recorded and reported.

Populations of bone marrow colony forming cells were evaluated. Bone marrow was collected and the stem cell assay conducted, as follows: one femur per mouse was cut at the distal and proximal ends. Using a syringe and 25 gauge needle, 5 mL of IMDM media was flushed through the femur into a collection tube (2.5 mL flushed into one end, then 2.5 ml into the other end). The tube was washed using Iscove's Modified Dulbecco's Media containing 20% fetal bovine serum and the cell concentration was adjusted to the desired count for the colony assay.

In triplicate, the marrow cells were put into a mixture of methylcellulose and plated into 35 mm diameter dishes. The dishes were incubated for approximately 2 weeks (37°C, 5% $CO_2$, 100% relative humidity) then manually scored under a microscope for myeloid, erythroid, and mixed colonies.

Data generated included the following:

| | |
|---|---|
| Body weights: | Initial and daily |
| Hematology: | Blood collected prior to necropsy |
| Bone marrow: | Bone marrow count, Myeloid/Erythroid ratio, Stem cell population |
| Food consumption: | Daily |

Statistical tests were performed at the α level of 0.01. Listing of the individual data and summary statistics (mean, standard error and sample size) was provided. The average body weight changes from study day 3 for study days 4-10 and 11-17 and average food consumption for study days 4-10 and 11-17 was analyzed. Since the groups consist of various dosages of AZT and Procysteine®, appropriate dosage response models were established. Specifically, the interaction of AZT and Procysteine® was evaluated. In other words, the dosage response of Procysteine® was estimated and compared for each dosage of AZT.

The resultant data is set forth in Tables 3-9 that follow.

Total Proliferating (%)

| AZT<br>Procysteine | 0 mg/kg<br>0.0% | 0 mg/kg<br>1.0% | 50 mg/kg<br>0.0% | 500 mg/kg<br>0.0% | 50 mg/kg<br>1.0% | 500 mg/kg<br>1.0% | AZT Dose<br>-0.0% | Response at<br>-1.0% |
|---|---|---|---|---|---|---|---|---|
| **Male** | | | | | | | | |
| Mean | 21.3 | 21.4 | 29.8 | 28.7 | 22.7 | 23.5 | | |
| Std. Err. | 0.3 | 0.2 | 0.3 | 0.4 | 0.3 | 0.3 | | |
| N | 10 | 10 | 10 | 10 | 10 | 10 | | |
| **Female** | | | | | | | | |
| Mean | 20.2 | 20.9 | 28.0 | 28.9 | 21.1 | 22.0 | | |
| Std. Err. | 0.3 | 0.4 | 0.2 | 0.2 | 0.4 | 0.3 | | |
| N | 10 | 8 | 10 | 10 | 8 | 9 | | |
| **Combined** | | | | | | | | |
| Mean | 20.7 | 21.2 | 28.9 | 28.8 | 22.0 | 22.8 | * | * |
| Std. Err. | 0.2 | 0.2 | 0.3 | 0.2 | 0.3 | 0.3 | | |
| N | 20 | 18 | 20 | 20 | 18 | 19 | | |

Table 3: Bone Marrow-Myeloid Series
Listing of Mean and Standard Error; * Indicates Significance at Alpha = 0.01
-Indicates Not Significant, Blank Indicates No Statistical Test Performed

Basophil (%)

| AZT<br>Procysteine | 0 mg/kg<br>0.0% | 0 mg/kg<br>1.0% | 50 mg/kg<br>0.0% | 500 mg/kg<br>0.0% | 50 mg/kg<br>1.0% | 500 mg/kg<br>1.0% | AZT Dose<br>-0.0% | Response at<br>-1.0% |
|---|---|---|---|---|---|---|---|---|
| **Male** | | | | | | | | |
| Mean | 0.0 | 0.0 | 0.0 | 0.0 | 0.0 | 0.0 | | |
| Std. Err. | 0.0 | 0.0 | 0.0 | 0.0 | 0.0 | 0.0 | | |
| N | 10 | 10 | 10 | 10 | 10 | 10 | | |
| **Female** | | | | | | | | |
| Mean | 0.0 | 0.0 | 0.0 | 0.0 | 0.0 | 0.0 | | |
| Std. Err. | 0.0 | 0.0 | 0.0 | 0.0 | 0.0 | 0.0 | | |
| N | 10 | 8 | 10 | 10 | 8 | 9 | | |
| **Combined** | | | | | | | | |
| Mean | 0.0 | 0.0 | 0.0 | 0.0 | 0.0 | 0.0 | - | - |
| Std. Err. | 0.0 | 0.0 | 0.0 | 0.0 | 0.0 | 0.0 | | |
| N | 20 | 18 | 20 | 20 | 18 | 19 | | |

Total Myeloid (%)

| AZT<br>Procysteine | 0 mg/kg<br>0.0% | 0 mg/kg<br>1.0% | 50 mg/kg<br>0.0% | 500 mg/kg<br>0.0% | 50 mg/kg<br>1.0% | 500 mg/kg<br>1.0% | AZT Dose<br>-0.0% | Response at<br>-1.0% |
|---|---|---|---|---|---|---|---|---|
| **Male** | | | | | | | | |
| Mean | 62.4 | 62.9 | 67.2 | 67.0 | 63.6 | 64.9 | | |
| Std. Err. | 0.3 | 0.2 | 0.2 | 0.3 | 0.3 | 0.2 | | |
| N | 10 | 10 | 10 | 10 | 10 | 10 | | |
| **Female** | | | | | | | | |
| Mean | 60.8 | 62.8 | 67.4 | 66.3 | 63.1 | 63.3 | | |
| Std. Err. | 0.3 | 0.4 | 0.2 | 0.2 | 0.4 | 0.3 | | |
| N | 10 | 8 | 10 | 10 | 8 | 9 | | |
| **Combined** | | | | | | | | |
| Mean | 61.6 | 62.9 | 67.3 | 66.7 | 63.3 | 64.1 | * | * |
| Std. Err. | 0.3 | 0.2 | 0.2 | 0.2 | 0.2 | 0.2 | | |
| N | 20 | 18 | 20 | 20 | 18 | 19 | | |

Table 4: Bone Marrow-Erythroid Series

Listing of Mean and Standard Error; * Indicates Significance at Alpha = 0.1

-Indicates Not Significant, Blank Indicates No Statistical Test Performed

Rubricyte (%)

| AZT<br>Procysteine | 0 mg/kg<br>0.0% | 0 mg/kg<br>1.0% | 50 mg/kg<br>0.0% | 500 mg/kg<br>0.0% | 50 mg/kg<br>1.0% | 500 mg/kg<br>1.0% | AZT Dose<br>-0.0% | Response at<br>-1.0% |
|---|---|---|---|---|---|---|---|---|
| **Male** | | | | | | | | |
| Mean | 22.0 | 222.7 | 19.9 | 20.2 | 23.6 | 22.8 | * | - |
| Std. Err. | 0.3 | 0.2 | 0.1 | 0.3 | 0.2 | 0.2 | | |
| N | 10 | 10 | 10 | 10 | 10 | 10 | | |
| **Female** | | | | | | | | |
| Mean | 23.4 | 22.2 | 19.9 | 20.0 | 22.2 | 21.6 | * | - |
| Std. Err. | 0.2 | 0.4 | 0.2 | 0.1 | 0.3 | 0.3 | | |
| N | 10 | 8 | 10 | 10 | 8 | 9 | | |
| **Combined** | | | | | | | | |
| Mean | 22.7 | 22.35 | 19.9 | 20.1 | 23.0 | 22.3 | | |
| Std. Err. | 0.3 | 0.2 | 0.1 | 0.2 | 0.2 | 0.2 | | |
| N | 20 | 18 | 20 | 20 | 18 | 19 | | |

Total Proliferating (%)

| AZT<br>Procysteine | 0 mg/kg<br>0.0% | 0 mg/kg<br>1.0% | 50 mg/kg<br>0.0% | 500 mg/kg<br>0.0% | 50 mg/kg<br>1.0% | 500 mg/kg<br>1.0% | AZT Dose<br>-0.0% | Response at<br>-1.0% |
|---|---|---|---|---|---|---|---|---|
| **Male** | | | | | | | | |
| Mean | 31.0 | 31.7 | 30.5 | 30.3 | 34.0 | 32.8 | | |
| Std. Err. | 0.3 | 0.4 | 0.1 | 0.3 | 0.4 | 0.2 | | |
| N | 10 | 10 | 10 | 10 | 10 | 10 | | |
| **Female** | | | | | | | | |
| Mean | 33.3 | 31.3 | 29.2 | 31.3 | 31.3 | 31.5 | | |
| Std. Err. | 0.5 | 0.3 | 0.2 | 0.2 | 0.2 | 0.2 | | |
| N | 10 | 8 | 10 | 10 | 8 | 9 | | |
| **Combined** | | | | | | | | |
| Mean | 32.6 | 31.5 | 29.9 | 30.8 | 32.8 | 32.2 | * | - |
| Std. Err. | 0.3 | 0.2 | 0.2 | 0.2 | 0.4 | 0.2 | | |
| N | 20 | 18 | 20 | 20 | 18 | 19 | | |

Table 4: Bone Marrow-Erythroid Series

Listing of Mean and Standard Error; * Indicates Significance at Alpha = 0.01

- Indicates Not Significant, Blank Indicates No Statistical Test Performed

Metarubricyte (%)

| AZT<br>Procysteine | 0 mg/kg<br>0.0% | 0 mg/kg<br>1.0% | 50 mg/kg<br>0.0% | 500 mg/kg<br>0.0% | 50 mg/kg<br>1.0% | 500 mg/kg<br>1.0% | AZT Dose<br>-0.0% | Response at<br>-1.0% |
|---|---|---|---|---|---|---|---|---|
| **Male** | | | | | | | | |
| Mean | 4.3 | 4.0 | 1.0 | 1.5 | 1.0 | 1.0 | * | * |
| Std. Err. | 0.2 | 0.2 | 0.1 | 0.1 | 0.1 | 0.1 | | |
| N | 10 | 10 | 10 | 10 | 10 | 10 | | |

| | | | | | | | | |
|---|---|---|---|---|---|---|---|---|
| **Female** | | | | | | | | |
| Mean | 4.3 | 4.2 | 2.2 | 1.2 | 4.0 | 3.8 | * | - |
| Std. Err. | 0.3 | 0.2 | 0.2 | 0.1 | 0.2 | 0.2 | | |
| N | 10 | 8 | 10 | 10 | 8 | 9 | | |
| **Combined** | | | | | | | | |
| Mean | 4.4 | 4.1 | 1.6 | 1.3 | 2.3 | 2.4 | | |
| Std. Err. | 0.2 | 0.1 | 0.2 | 0.1 | 0.4 | 0.3 | | |
| N | 20 | 18 | 20 | 20 | 18 | 19 | | |

Total Erythroid (%)

| AZT | 0 mg/kg | 0 mg/kg | 50 mg/kg | 500 mg/kg | 50 mg/kg | 500 mg/kg | AZT Dose | Response at |
|---|---|---|---|---|---|---|---|---|
| Procysteine | 0.0% | 1.0% | 0.0% | 0.0% | 1.0% | 1.0% | -0.0% | -1.0% |
| **Male** | | | | | | | | |
| Mean | 36.3 | 35.7 | 31.5 | 31.8 | 35.0 | 33.9 | | |
| Std. Err. | 0.3 | 0.3 | 0.1 | 0.3 | 0.3 | 0.2 | | |
| N | 10 | 10 | 10 | 10 | 10 | 10 | | |
| **Female** | | | | | | | | |
| Mean | 37.6 | 35.4 | 31.4 | 32.4 | 35.2 | 35.4 | | |
| Std. Err. | 0.3 | 0.1 | 0.3 | 0.2 | 0.2 | 0.2 | | |
| N | 10 | 8 | 10 | 10 | 8 | 9 | | |
| **Combined** | | | | | | | | |
| Mean | 37.0 | 35.6 | 31.5 | 37.1 | 35.1 | 34.6 | * | - |
| Std. Err. | 0.3 | 0.2 | 0.2 | 0.2 | 0.2 | 0.2 | | |
| N | 20 | 18 | 20 | 20 | 18 | 19 | | |

Table 5: Blood Assays

Listing of Mean and Standard Error; * Indicates Significance at Alpha = 0.01

- Indicates Not Significant, Blank Indicates No Statistical Test Performed

WBC (x10**3/ul)

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT Dose -0.0% | Response at -1.0% |
|---|---|---|---|---|---|---|---|---|
| **Male** | | | | | | | | |
| Mean | 1.7 | 1.9 | 1.6 | 2.3 | 1.6 | 2.1 | | |
| Std. Err. | 0.3 | 0.2 | 0.2 | 0.3 | 0.1 | 0.2 | | |
| N | 10 | 10 | 10 | 10 | 10 | 10 | | |
| **Female** | | | | | | | | |
| Mean | 2.1 | 1.6 | 1.7 | 2.3 | 2.0 | 1.6 | | |
| Std. Err. | 0.2 | 0.1 | 0.2 | 0.2 | 0.2 | 0.2 | | |
| N | 10 | 9 | 10 | 10 | 9 | 10 | | |
| **Combined** | | | | | | | | |
| Mean | 1.9 | 1.7 | 1.7 | 2.3 | 1.8 | 1.9 | - | - |
| Std. Err. | 0.2 | 0.1 | 0.1 | 0.2 | 0.1 | 0.1 | | |
| N | 20 | 19 | 20 | 20 | 19 | 20 | | |

RBC (x10**6/UL)

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT Dose -0.0% | Response at -1.0% |
|---|---|---|---|---|---|---|---|---|
| **Male** | | | | | | | | |
| Mean | 7.46 | 7.46 | 6.95 | 6.18 | 7.13 | 6.19 | | |
| Std. Err. | 0.06 | 0.03 | 0.05 | 0.08 | 0.08 | 0.11 | | |
| N | 10 | 10 | 10 | 10 | 10 | 10 | | |
| **Female** | | | | | | | | |
| Mean | 7.25 | 7.36 | 6.77 | 6.32 | 6.99 | 6.29 | | |
| Std. Err. | 0.07 | 0.06 | 0.04 | 0.10 | 0.10 | 0.05 | | |
| N | 10 | 9 | 10 | 10 | 9 | 10 | | |
| **Combined** | | | | | | | | |
| Mean | 7.36 | 7.41 | 6.86 | 6.20 | 7.07 | 6.24 | * | * |
| Std. Err. | 0.05 | 0.04 | 0.04 | 0.07 | 0.06 | 0.06 | | |
| N | 20 | 19 | 20 | 20 | 19 | 20 | | |

Table 5: Blood Assays

Listing of Mean and Standard Error; * Indicates Significance at Alpha = 0.01

- Indicates Not Significant, Blank Indicates No Statistical Test Performed

HGB (g/L)

| AZT | 0 mg/kg | 0 mg/kg | 50 mg/kg | 500 mg/kg | 50 mg/kg | 500 mg/kg | AZT Dose | Response at |
|---|---|---|---|---|---|---|---|---|
| Procysteine | 0.0% | 1.0% | 0.0% | 0.0% | 1.0% | 1.0% | -0.0% | -1.0% |
| **Male** | | | | | | | | |
| Mean | 13.2 | 13.2 | 12.6 | 11.4 | 13.0 | 11.5 | | |
| Std. Err. | 0.1 | 0.1 | 0.1 | 0.2 | 0.1 | 0.2 | | |
| N | 10 | 10 | 10 | 10 | 10 | 10 | | |
| **Female** | | | | | | | | |
| Mean | 13.0 | 13.0 | 12.5 | 11.7 | 12.9 | 11.7 | | |
| Std. Err. | 0.1 | 0.1 | 0.1 | 0.2 | 0.2 | 0.2 | | |
| N | 10 | 9 | 10 | 10 | 9 | 10 | | |
| **Combined** | | | | | | | | |
| Mean | 13.1 | 13.1 | 12.5 | 11.6 | 12.9 | 11.6 | * | * |
| Std. Err. | 0.1 | 0.1 | 0.1 | 0.1 | 0.1 | 0.1 | | |
| N | 20 | 19 | 20 | 20 | 19 | 20 | | |

HCT (%)

| AZT | 0 mg/kg | 0 mg/kg | 50 mg/kg | 500 mg/kg | 50 mg/kg | 500 mg/kg | AZT Dose | Response at |
|---|---|---|---|---|---|---|---|---|
| Procysteine | 0.0% | 1.0% | 0.0% | 0.0% | 1.0% | 1.0% | -0.0% | -1.0% |
| **Male** | | | | | | | | |
| Mean | 37.4 | 37.0 | 36.0 | 33.0 | 36.8 | 33.2 | | |
| Std. Err. | 0.4 | 0.3 | 0.3 | 0.5 | 0.5 | 0.7 | | |
| N | 10 | 10 | 10 | 10 | 10 | 10 | | |
| **Female** | | | | | | | | |
| Mean | 36.1 | 35.9 | 34.4 | 32.1 | 35.2 | 32.5 | | |
| Std. Err. | 0.4 | 0.4 | 0.3 | 0.5 | 0.5 | 0.5 | | |
| N | 10 | 9 | 10 | 10 | 9 | 10 | | |
| **Combined** | | | | | | | | |
| Mean | 36.8 | 36.5 | 35.2 | 32.5 | 36.0 | 32.8 | * | * |
| Std. Err. | 0.3 | 0.2 | 0.3 | 0.4 | 0.4 | 0.4 | | |
| N | 20 | 19 | 20 | 20 | 19 | 20 | | |

Table 5: Blood Assays

Listing of Mean and Standard Error; * Indicates Significance at Alpha = 0.01

- Indicates Not Significant, Blank Indicates No Statistical Test Performed

MCY (f/L)

| AZT | 0 mg/kg | 0 mg/kg | 50 mg/kg | 500 mg/kg | 50 mg/kg | 500 mg/kg | AZT Dose | Response at |
|---|---|---|---|---|---|---|---|---|
| Procysteine | 0.0% | 1.0% | 0.0% | 0.0% | 1.0% | 1.0% | -0.0% | -1.0% |
| **Male** | | | | | | | | |
| Mean | 51 | 51 | 52 | 54 | 52 | 54 | | |
| Std. Err. | 0 | 0 | 0 | 0 | 0 | 0 | | |
| N | 10 | 10 | 10 | 10 | 10 | 10 | | |

| | | | | | | | | |
|---|---|---|---|---|---|---|---|---|
| **Female** | | | | | | | | |
| Mean | 50 | 49 | 51 | 52 | 51 | 52 | | |
| Std. Err. | 0 | 0 | 0 | 0 | 0 | 1 | | |
| N | 10 | 9 | 10 | 10 | 9 | 10 | | |
| **Combined** | | | | | | | | |
| Mean | 51 | 50 | 52 | 53 | 52 | 53 | * | * |
| Std. Err. | 0 | 0 | 0 | 0 | 0 | 0 | | |
| N | 20 | 19 | 20 | 20 | 19 | 20 | | |

| | MCH (pw) | | | | | | | |
|---|---|---|---|---|---|---|---|---|
| AZT | 0 mg/kg | 0 mg/kg | 50 mg/kg | 500 mg/kg | 50 mg/kg | 500 mg/kg | AZT Dose | Response at |
| Procysteine | 0.0% | 1.0% | 0.0% | 0.0% | 1.0% | 1.0% | -0.0% | -1.0% |
| **Male** | | | | | | | | |
| Mean | 17.6 | 17.7 | 18.2 | 18.6 | 18.3 | 18.6 | | |
| Std. Err. | 0.1 | 0.1 | 0.1 | 0.2 | 0.1 | 0.1 | | |
| N | 10 | 10 | 10 | 10 | 10 | 10 | | |
| **Female** | | | | | | | | |
| Mean | 18.0 | 17.6 | 18.4 | 18.8 | 18.4 | 18.6 | | |
| Std. Err. | 0.2 | 0.1 | 0.1 | 0.2 | 0.2 | 0.2 | | |
| N | 10 | 9 | 10 | 10 | 9 | 10 | | |
| **Combined** | | | | | | | | |
| Mean | 17.8 | 17.7 | 18.3 | 18.7 | 18.3 | 18.6 | * | * |
| Std. Err. | 0.1 | 0.1 | 0.1 | 0.1 | 0.1 | 0.1 | | |
| N | 20 | 19 | 20 | 20 | 19 | 20 | | |

Table 5: Blood Assays

Listing of Mean and Standard Error; * Indicates Significance at Alpha = 0.01

- Indicates Not Significant, Blank Indicates No Statistical Test Performed

WBBC (/100 WBC)

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT Dose -0.0% | Response at -1.0% |
|---|---|---|---|---|---|---|---|---|
| **Male** | | | | | | | | |
| Mean | 0 | 0 | 0 | 0 | 0 | 0 | | |
| Std. Err. | 0 | 0 | 0 | 0 | 0 | 0 | | |
| N | 10 | 10 | 10 | 10 | 10 | 10 | | |
| **Female** | | | | | | | | |
| Mean | 0 | 0 | 0 | 0 | 0 | 0 | | |
| Std. Err. | 0 | 0 | 0 | 0 | 0 | 0 | | |
| N | 10 | 9 | 10 | 10 | 9 | 10 | | |
| **Combined** | | | | | | | | |
| Mean | 0 | 0 | 0 | 0 | 0 | 0 | - | - |
| Std. Err. | 0 | 0 | 0 | 0 | 0 | 0 | | |
| N | 20 | 19 | 20 | 20 | 19 | 20 | | |

Aniso (coded)

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT Dose -0.0% | Response at -1.0% |
|---|---|---|---|---|---|---|---|---|
| **Male** | | | | | | | | |
| Mean | 0.2 | 0.1 | 0.3 | 0.7 | 0.1 | 0.5 | | |
| Std. Err. | 0.1 | 0.1 | 0.1 | 0.1 | 0.1 | 0.1 | | |
| N | 10 | 10 | 10 | 10 | 10 | 10 | | |
| **Female** | | | | | | | | |
| Mean | 0.1 | 0.1 | 0.2 | 0.7 | 0.1 | 0.5 | | |
| Std. Err. | 0.1 | 0.1 | 0.1 | 0.1 | 0.1 | 0.1 | | |
| N | 10 | 9 | 10 | 10 | 9 | 10 | | |
| **Combined** | | | | | | | | |
| Mean | 0.1 | 0.1 | 0.2 | 0.7 | 0.1 | 0.5 | * | * |
| Std. Err. | 0.0 | 0.0 | 0.1 | 0.1 | 0.0 | 0.1 | | |
| N | 20 | 19 | 20 | 20 | 19 | 20 | | |

Table 5: Blood Assays

Listing of Mean and Standard Error; * Indicates Significance at Alpha = 0.01

- Indicates Not Significant, Blank Indicates No Statistical Test Performed

Poly (coded)

| | 0 | 0 | 50 | 500 | 50 | 500 | AZT | Response |
|---|---|---|---|---|---|---|---|---|
| AZT | mg/kg | mg/kg | mg/kg | mg/kg | mg/kg | mg/kg | Dose | at |
| Procysteine | 0.0% | 1.0% | 0.0% | 0.0% | 1.0% | 1.0% | -0.0% | -1.0% |
| Male | | | | | | | | |
| Mean | 0.7 | 0.8 | 0.9 | 1.0 | 0.7 | 0.8 | | |
| Std. Err. | 0.1 | 0.1 | 0.1 | 0.1 | 0.1 | 0.1 | | |
| N | 10 | 10 | 10 | 10 | 10 | 10 | | |
| Female | | | | | | | | |
| Mean | 0.6 | 0.6 | 0.7 | 0.8 | 0.8 | 0.8 | | |
| Std. Err. | 0.1 | 0.1 | 0.1 | 0.1 | 0.2 | 0.1 | | |
| N | 10 | 9 | 10 | 10 | 9 | 10 | | |
| Combined | | | | | | | | |
| Mean | 0.6 | 0.7 | 0.8 | 0.9 | 0.8 | 0.8 | * | - |
| Std. Err. | 0.0 | 0.1 | 0.1 | 0.0 | 0.1 | 0.1 | | |
| N | 20 | 19 | 20 | 20 | 19 | 20 | | |

Burr Cells (coded)

| | 0 | 0 | 50 | 500 | 50 | 500 | AZT | Response |
|---|---|---|---|---|---|---|---|---|
| AZT | mg/kg | mg/kg | mg/kg | mg/kg | mg/kg | mg/kg | Dose | at |
| Procysteine | 0.0% | 1.0% | 0.0% | 0.0% | 1.0% | 1.0% | -0.0% | -1.0% |
| Male | | | | | | | | |
| Mean | 0.1 | 0.2 | 0.1 | 0.0 | 0.0 | 0.0 | | |
| Std. Err. | 0.1 | 0.1 | 0.1 | 0.0 | 0.0 | 0.0 | | |
| N | 10 | 10 | 10 | 10 | 10 | 10 | | |
| Female | | | | | | | | |
| Mean | 0.1 | 0.0 | 0.1 | 0.1 | 0.1 | 0.2 | | |
| Std. Err. | 0.1 | 0.0 | 0.1 | 0.1 | 0.1 | 0.1 | | |
| N | 10 | 9 | 10 | 10 | 9 | 10 | | |
| Combined | | | | | | | | |
| Mean | 0.1 | 0.1 | 0.1 | 0.0 | 0.1 | 0.1 | - | - |
| Std. Err. | 0.1 | 0.0 | 0.0 | 0.0 | 0.0 | 0.0 | | |
| N | 20 | 19 | 20 | 20 | 19 | 20 | | |

Table 5: Blood Assays

Listing of Mean and Standard Error; * Indicates Significance at Alpha = 0.01

- Indicates Not Significant, Blank Indicates No Statistical Test Performed

Howell Jolly Bodies (coded)

| | 0 | 0 | 50 | 500 | 50 | 500 | AZT | Response |
|---|---|---|---|---|---|---|---|---|
| AZT | mg/kg | mg/kg | mg/kg | mg/kg | mg/kg | mg/kg | Dose | at |
| Procysteine | 0.0% | 1.0% | 0.0% | 0.0% | 1.0% | 1.0% | -0.0% | -1.0% |
| Male | | | | | | | | |
| Mean | 0.1 | 0.1 | 0.1 | 0.5 | 0.1 | 0.3 | | |
| Std. Err. | 0.1 | 0.1 | 0.1 | 0.1 | 0.1 | 0.1 | | |
| N | 10 | 10 | 10 | 10 | 10 | 10 | | |

| | | | | | | | | |
|---|---|---|---|---|---|---|---|---|
| **Female** | | | | | | | | |
| Mean | 0.0 | 0.0 | 0.0 | 0.3 | 0.1 | 0.2 | | |
| Std. Err. | 0.0 | 0.0 | 0.0 | 0.1 | 0.1 | 0.1 | | |
| N | 10 | 9 | 10 | 10 | 9 | 10 | | |
| **Combined** | | | | | | | | |
| Mean | 0.1 | 0.0 | 0.0 | 0.4 | 0.1 | 0.2 | * | - |
| Std. Err. | 0.0 | 0.0 | 0.0 | 0.1 | 0.0 | 0.1 | | |
| N | 20 | 19 | 20 | 20 | 19 | 20 | | |

Reticulocytes (%)

| AZT<br>Procysteine | 0 mg/kg<br>0.0% | 0 mg/kg<br>1.0% | 50 mg/kg<br>0.0% | 500 mg/kg<br>0.0% | 50 mg/kg<br>1.0% | 500 mg/kg<br>1.0% | AZT Dose<br>-0.0% | Response at<br>-1.0% |
|---|---|---|---|---|---|---|---|---|
| **Male** | | | | | | | | |
| Mean | 1.4 | 1.5 | 1.5 | 1.7 | 1.4 | 1.6 | | |
| Std. Err. | 0.1 | 0.2 | 0.1 | 0.1 | 0.2 | 0.1 | | |
| N | 10 | 10 | 10 | 10 | 10 | 10 | | |
| **Female** | | | | | | | | |
| Mean | 1.3 | 1.4 | 1.4 | 1.3 | 1.5 | 1.3 | | |
| Std. Err. | 0.1 | 0.1 | 0.2 | 0.2 | 0.2 | 0.2 | | |
| N | 10 | 9 | 10 | 10 | 9 | 9 | | |
| **Combined** | | | | | | | | |
| Mean | 1.4 | 1.5 | 1.4 | 1.5 | 1.4 | 1.4 | - | - |
| Std. Err. | 0.1 | 0.1 | 0.1 | 0.1 | 0.1 | 0.1 | | |
| N | 20 | 19 | 20 | 20 | 19 | 19 | | |

Table 6: Bone Marrow-Myeloid Series

Listing of Mean and Standard Error; * Indicates Significance at Alpha = 0.01

- Indicates Not Significant, Blank Indicates No Statistical Test Performed

Myeloblast (%)

| AZT<br>Procysteine | 0 mg/kg<br>0.0% | 0 mg/kg<br>1.0% | 50 mg/kg<br>0.0% | 500 mg/kg<br>0.0% | 50 mg/kg<br>1.0% | 500 mg/kg<br>1.0% | AZT Dose<br>-0.0% | Response at<br>-1.0% |
|---|---|---|---|---|---|---|---|---|
| **Male** | | | | | | | | |
| Mean | 3.0 | 3.3 | 3.8 | 3.5 | 3.5 | 3.6 | * | - |
| Std. Err. | 0.2 | 0.2 | 0.1 | 0.1 | 0.2 | 0.1 | | |
| N | 10 | 10 | 10 | 10 | 10 | 10 | | |
| **Female** | | | | | | | | |
| Mean | 2.4 | 3.2 | 3.5 | 3.6 | 2.7 | 3.1 | * | - |
| Std. Err. | 0.0 | 0.0 | 0.0 | 0.1 | 0.1 | 0.1 | | |
| N | 10 | 8 | 10 | 10 | 8 | 10 | | |
| **Combined** | | | | | | | | |
| Mean | 2.7 | 3.2 | 3.6 | 3.6 | 3.1 | 3.3 | | |
| Std. Err. | 0.1 | 0.1 | 0.1 | 0.1 | 0.2 | 0.1 | | |
| N | 20 | 18 | 20 | 20 | 18 | 19 | | |

Promyelocyte (%)

| AZT<br>Procysteine | 0 mg/kg<br>0.0% | 0 mg/kg<br>1.0% | 50 mg/kg<br>0.0% | 500 mg/kg<br>0.0% | 50 mg/kg<br>1.0% | 500 mg/kg<br>1.0% | AZT Dose<br>-0.0% | Response at<br>-1.0% |
|---|---|---|---|---|---|---|---|---|
| **Male** | | | | | | | | |
| Mean | 9.3 | 8.5 | 13.0 | 12.7 | 9.6 | 9.9 | | |
| Std. Err. | 0.2 | 0.2 | 0.2 | 0.3 | 0.1 | 0.1 | | |
| N | 10 | 10 | 10 | 10 | 10 | 10 | | |
| **Female** | | | | | | | | |
| Mean | 9.0 | 8.4 | 12.1 | 12.5 | 8.9 | 9.1 | | |
| Std. Err. | 0.2 | 0.3 | 0.1 | 0.1 | 0.3 | 0.2 | | |
| N | 10 | 8 | 10 | 10 | 8 | 9 | | |
| **Combined** | | | | | | | | |
| Mean | 9.1 | 0.4 | 12.6 | 12.6 | 9.3 | 9.5 | * | * |
| Std. Err. | 0.2 | 0.2 | 0.2 | 0.2 | 0.2 | 0.1 | | |
| N | 20 | 18 | 20 | 20 | 18 | 19 | | |

Table 6: Bone Marrow-Myeloid Series

Listing of Mean and Standard Error; * Indicates Significance at Alpha = 0.01

- Indicates Not Significant, Blank Indicates No Statistical Test Performed

Myelocyte (%)

| AZT<br>Procysteine | 0<br>mg/kg<br>0.0% | 0<br>mg/kg<br>1.0% | 50<br>mg/kg<br>0.0% | 500<br>mg/kg<br>0.0% | 50<br>mg/kg<br>1.0% | 500<br>mg/kg<br>1.0% | AZT<br>Dose<br>-0.0% | Response<br>at<br>-1.0% |
|---|---|---|---|---|---|---|---|---|
| **Male** | | | | | | | | |
| Mean | 9.1 | 9.6 | 12.9 | 12.4 | 9.6 | 10.0 | | |
| Std. Err. | 0.3 | 0.3 | 0.2 | 0.3 | 0.2 | 0.3 | | |
| N | 10 | 10 | 10 | 10 | 10 | 10 | | |
| **Female** | | | | | | | | |
| Mean | 8.8 | 9.4 | 12.5 | 12.7 | 9.6 | 9.8 | | |
| Std. Err. | 0.2 | 0.7 | 0.3 | 0.2 | 0.2 | 0.3 | | |
| N | 10 | 8 | 10 | 10 | 8 | 9 | | |
| **Combined** | | | | | | | | |
| Mean | 9.0 | 9.5 | 12.7 | 12.6 | 9.6 | 9.9 | * | - |
| Std. Err. | 0.2 | 0.2 | 0.2 | 0.2 | 0.1 | 0.2 | | |
| N | 20 | 18 | 20 | 20 | 18 | 19 | | |

Metacyclocyte (%)

| AZT<br>Procysteine | 0<br>mg/kg<br>0.0% | 0<br>mg/kg<br>1.0% | 50<br>mg/kg<br>0.0% | 500<br>mg/kg<br>0.0% | 50<br>mg/kg<br>1.0% | 500<br>mg/kg<br>1.0% | AZT<br>Dose<br>-0.0% | Response<br>at<br>-1.0% |
|---|---|---|---|---|---|---|---|---|
| **Male** | | | | | | | | |
| Mean | 8.8 | 8.0 | 8.7 | 8.6 | 9.8 | 9.0 | - | * |
| Std. Err. | 0.3 | 0.3 | 0.2 | 0.2 | 0.3 | 0.2 | | |
| N | 10 | 10 | 10 | 10 | 10 | 10 | | |
| **Female** | | | | | | | | |
| Mean | 0.4 | 8.5 | 8.9 | 9.2 | 8.8 | 9.9 | - | * |
| Std. Err. | 0.1 | 0.4 | 0.2 | 0.2 | 0.4 | 0.2 | | |
| N | 10 | 8 | 10 | 10 | 8 | 9 | | |
| **Combined** | | | | | | | | |
| Mean | 8.6 | 8.2 | 8.8 | 8.9 | 9.3 | 9.4 | | |
| Std. Err. | 0.1 | 0.2 | 0.1 | 0.2 | 0.2 | 0.2 | | |
| N | 20 | 18 | 20 | 20 | 18 | 19 | | |

Table 6: Bone Marrow-Myeloid Series

Listing of Mean and Standard Error; * Indicates Significance at Alpha = 0.01

- Indicates Not Significant, Blank Indicates No statistical Test Performed

Band (%)

| AZT<br>Procysteine | 0<br>mg/kg<br>0.0% | 0<br>mg/kg<br>1.0% | 50<br>mg/kg<br>0.0% | 500<br>mg/kg<br>0.0% | 50<br>mg/kg<br>1.0% | 500<br>mg/kg<br>1.0% | AZT<br>Dose<br>-0.0% | Response<br>at<br>-1.0% |
|---|---|---|---|---|---|---|---|---|
| **Male** | | | | | | | | |
| Mean | 15.2 | 15.8 | 13.6 | 14.0 | 15.2 | 15.5 | | |
| Std. Err. | 0.3 | 0.2 | 0.2 | 0.2 | 0.1 | 0.2 | | |
| N | 10 | 10 | 10 | 10 | 10 | 10 | | |

| | | | | | | | | |
|---|---|---|---|---|---|---|---|---|
| Female | | | | | | | | |
| Mean | 15.6 | 15.5 | 14.9 | 13.7 | 15.3 | 15.7 | | |
| Std. Err. | 0.1 | 0.3 | 0.2 | 0.3 | 0.3 | 0.3 | | |
| N | 10 | 8 | 10 | 10 | 8 | 9 | | |
| Combined | | | | | | | | |
| Mean | 15.4 | 15.6 | 14.2 | 13.8 | 15.5 | 15.6 | * | - |
| Std. Err. | 0.2 | 0.2 | 0.2 | 0.2 | 0.1 | 0.1 | | |
| N | 20 | 18 | 20 | 20 | 18 | 19 | | |

Neutrophil (%)

| AZT<br>Procysteine | 0 mg/kg<br>0.0% | 0 mg/kg<br>1.0% | 50 mg/kg<br>0.0% | 500 mg/kg<br>0.0% | 50 mg/kg<br>1.0% | 500 mg/kg<br>1.0% | AZT Dose<br>-0.0% | Response at<br>-1.0% |
|---|---|---|---|---|---|---|---|---|
| Male | | | | | | | | |
| Mean | 13.4 | 13.4 | 12.3 | 12.9 | 12.9 | 13.5 | * | - |
| Std. Err. | 0.3 | 0.3 | 0.2 | 0.1 | 0.2 | 0.2 | | |
| N | 10 | 10 | 10 | 10 | 10 | 10 | | |
| Female | | | | | | | | |
| Mean | 13.2 | 13.7 | 12.5 | 12.4 | 13.6 | 12.4 | * | * |
| Std. Err. | 0.1 | 0.3 | 0.2 | 0.3 | 0.3 | 0.2 | | |
| N | 10 | 8 | 10 | 10 | 8 | 9 | | |
| Combined | | | | | | | | |
| Mean | 13.3 | 13.5 | 12.4 | 12.6 | 13.2 | 13.0 | | |
| Std. Err. | 0.2 | 0.2 | 0.1 | 0.2 | 0.2 | 0.2 | | |
| N | 20 | 18 | 20 | 20 | 18 | 19 | | |

Table 6: Bone Marrow-Myeloid Series
Listing of Mean and Standard Error; * Indicates Significance at Alpha = 0.01
- Indicates Not Significant, Blank Indicates No Statistical Test Performed

| | Casinophil (%) | | | | | | | |
|---|---|---|---|---|---|---|---|---|
| AZT | 0 mg/kg | 0 mg/kg | 50 mg/kg | 500 mg/kg | 50 mg/kg | 500 mg/kg | AZT Dose | Response at |
| Procysteine | 0.0% | 1.0% | 0.0% | 0.0% | 1.0% | 1.0% | -0.0% | -1.0% |
| **Male** | | | | | | | | |
| Mean | 3.7 | 4.4 | 2.8 | 2.9 | 2.9 | 3.5 | | |
| Std. Err. | 0.2 | 0.2 | 0.1 | 0.2 | 0.2 | 0.2 | | |
| N | 10 | 10 | 10 | 10 | 10 | 10 | | |
| **Female** | | | | | | | | |
| Mean | 3.4 | 4.3 | 3.0 | 2.2 | 4.2 | 3.2 | | |
| Std. Err. | 0.2 | 0.1 | 0.2 | 0.1 | 0.2 | 0.3 | | |
| N | 10 | 8 | 10 | 10 | 8 | 9 | | |
| **Combined** | | | | | | | | |
| Mean | 3.5 | 4.4 | 2.9 | 2.6 | 3.5 | 3.3 | * | * |
| Std. Err. | 0.2 | 0.1 | 0.1 | 0.1 | 0.2 | 0.2 | | |
| N | 20 | 18 | 20 | 20 | 18 | 19 | | |

| | Basophil (%) | | | | | | | |
|---|---|---|---|---|---|---|---|---|
| AZT | 0 mg/kg | 0 mg/kg | 50 mg/kg | 500 mg/kg | 50 mg/kg | 500 mg/kg | AZT Dose | Response at |
| Procysteine | 0.0% | 1.0% | 0.0% | 0.0% | 1.0% | 1.0% | -0.0% | -1.0% |
| **Male** | | | | | | | | |
| Mean | 0.0 | 0.0 | 0.0 | 0.0 | 0.0 | 0.0 | | |
| Std. Err. | 0.0 | 0.0 | 0.0 | 0.0 | 0.0 | 0.0 | | |
| N | 10 | 10 | 10 | 10 | 10 | 10 | | |
| **Female** | | | | | | | | |
| Mean | 0.0 | 0.0 | 0.0 | 0.0 | 0.0 | 0.0 | | |
| Std. Err. | 0.0 | 0.0 | 0.0 | 0.0 | 0.0 | 0.0 | | |
| N | 10 | 8 | 10 | 10 | 8 | 9 | | |
| **Combined** | | | | | | | | |
| Mean | 0.0 | 0.0 | 0.0 | 0.0 | 0.0 | 0.0 | - | - |
| Std. Err. | 0.0 | 0.0 | 0.0 | 0.0 | 0.0 | 0.0 | | |
| N | 20 | 18 | 20 | 20 | 18 | 19 | | |

Table 6: Bone Marrow-Myeloid Series

Listing of Mean and Standard Error; * Indicates Significance at Alpha = 0.01

- Indicates Not Significant, Blank Indicates No Statistical Test Performed

Rubriblast (%)

| AZT | 0 mg/kg | 0 mg/kg | 50 mg/kg | 500 mg/kg | 50 mg/kg | 500 mg/kg | AZT Dose | Response at |
|---|---|---|---|---|---|---|---|---|
| Procysteine | 0.0% | 1.0% | 0.0% | 0.0% | 1.0% | 1.0% | -0.0% | -1.0% |
| Male | | | | | | | | |
| Mean | 1.1 | 1.1 | 0.7 | 0.7 | 1.1 | 1.0 | | |
| Std. Err. | 0.1 | 0.1 | 0.1 | 0.1 | 0.1 | 0.1 | | |
| N | 10 | 10 | 10 | 10 | 10 | 10 | | |
| Female | | | | | | | | |
| Mean | 1.1 | 1.0 | 0.5 | 0.6 | 1.1 | 1.1 | | |
| Std. Err. | 0.1 | 0.1 | 0.0 | 0.0 | 0.1 | 0.1 | | |
| N | 10 | 8 | 10 | 10 | 8 | 9 | | |
| Combined | | | | | | | | |
| Mean | 1.1 | 1.0 | 0.6 | 0.7 | 1.1 | 1.0 | * | - |
| Std. Err. | 0.0 | 0.0 | 0.0 | 0.0 | 0.0 | 0.0 | | |
| N | 20 | 18 | 20 | 20 | 18 | 19 | | |

Prerubricyte (%)

| AZT | 0 mg/kg | 0 mg/kg | 50 mg/kg | 500 mg/kg | 50 mg/kg | 500 mg/kg | AZT Dose | Response at |
|---|---|---|---|---|---|---|---|---|
| Procysteine | 0.0% | 1.0% | 0.0% | 0.0% | 1.0% | 1.0% | -0.0% | -1.0% |
| Male | | | | | | | | |
| Mean | 8.7 | 8.0 | 9.9 | 9.4 | 9.3 | 9.0 | | |
| Std. Err. | 0.3 | 0.2 | 0.2 | 0.2 | 0.2 | 0.2 | | |
| N | 10 | 10 | 10 | 10 | 10 | 10 | | |
| Female | | | | | | | | |
| Mean | 8.7 | 8.1 | 8.8 | 10.7 | 8.1 | 8.8 | | |
| Std. Err. | 0.3 | 0.3 | 0.2 | 0.3 | 0.2 | 0.4 | | |
| N | 10 | 8 | 10 | 10 | 8 | 9 | | |
| Combined | | | | | | | | |
| Mean | 8.7 | 8.0 | 9.3 | 10.0 | 8.7 | 8.9 | * | * |
| Std. Err. | 0.2 | 0.2 | 0.2 | 0.2 | 0.2 | 0.2 | | |
| N | 20 | 18 | 20 | 20 | 18 | 19 | | |

Table 7: Bone Marrow-Erythroid Series

Listing of Mean and Standard Error; * Indicates Significance at Alpha = 0.01

- Indicates Not Significant, Blank Indicates No Statistical Test Performed

Rubricyte (%)

| AZT | 0 mg/kg | 0 mg/kg | 50 mg/kg | 500 mg/kg | 50 mg/kg | 500 mg/kg | AZT Dose | Response at |
|---|---|---|---|---|---|---|---|---|
| Procysteine | 0.0% | 1.0% | 0.0% | 0.0% | 1.0% | 1.0% | -0.0% | -1.0% |
| Male | | | | | | | | |
| Mean | 22.0 | 22.7 | 19.9 | 20.2 | 23.6 | 22.8 | * | - |
| Std. Err. | 0.3 | 0.2 | 0.1 | 0.3 | 0.2 | 0.2 | | |
| N | 10 | 10 | 10 | 10 | 10 | 10 | | |

| | | | | | | | | |
|---|---|---|---|---|---|---|---|---|
| **Female** | | | | | | | | |
| Mean | 23.4 | 22.2 | 19.9 | 20.0 | 22.2 | 21.6 | * | - |
| Std. Err. | 0.2 | 0.4 | 0.2 | 0.1 | 0.3 | 0.3 | | |
| N | 10 | 8 | 10 | 10 | 8 | 9 | | |
| **Combined** | | | | | | | | |
| Mean | 22.7 | 22.5 | 19.9 | 20.1 | 23.0 | 22.3 | | |
| Std. Err. | 0.3 | 0.2 | 0.1 | 0.2 | 0.2 | 0.2 | | |
| N | 20 | 18 | 20 | 20 | 18 | 19 | | |

| | Metarubricyte (%) | | | | | | | |
|---|---|---|---|---|---|---|---|---|
| AZT | 0 mg/kg | 0 mg/kg | 50 mg/kg | 500 mg/kg | 50 mg/kg | 500 mg/kg | AZT Dose | Response at |
| Procysteine | 0.0% | 1.0% | 0.0% | 0.0% | 1.0% | 1.0% | -0.0% | -1.0% |
| **Male** | | | | | | | | |
| Mean | 4.5 | 4.0 | 1.0 | 1.5 | 1.0 | 1.0 | * | * |
| Std. Err. | 0.2 | 0.2 | 0.1 | 0.1 | 0.1 | 0.1 | | |
| N | 10 | 10 | 10 | 10 | 10 | 10 | | |
| **Female** | | | | | | | | |
| Mean | 4.3 | 4.2 | 2.2 | 1.2 | 4.0 | 3.8 | | |
| Std. Err. | 0.3 | 0.2 | 0.2 | 0.1 | 0.2 | 0.2 | | |
| N | 10 | 8 | 10 | 10 | 8 | 9 | | |
| **Combined** | | | | | | | | |
| Mean | 4.4 | 4.1 | 1.6 | 1.3 | 2.3 | 2.4 | | |
| Std. Err. | 0.2 | 0.1 | 0.2 | 0.1 | 0.4 | 0.3 | | |
| N | 20 | 18 | 20 | 20 | 18 | 19 | | |

Table 8: Bone Marrow-Miscellaneous Cells

Listing of Mean and Standard Error; * Indicates Significance at Alpha = 0.1

- Indicates Not Significant, Blank Indicates No Statistical Test Performed

| | Lymphocyte (%) | | | | | | | |
|---|---|---|---|---|---|---|---|---|
| AZT | 0 mg/kg | 0 mg/kg | 50 mg/kg | 500 mg/kg | 50 mg/kg | 500 mg/kg | AZT Dose | Response at |
| Procysteine | 0.0% | 1.0% | 0.0% | 0.0% | 1.0% | 1.0% | -0.0% | -1.0% |
| Male | | | | | | | | |
| Mean | 0.9 | 1.0 | 1.4 | 0.2 | 0.3 | 0.2 | * | * |
| Std. Err. | 0.1 | 0.1 | 0.1 | 0.1 | 0.1 | 0.1 | | |
| N | 10 | 10 | 10 | 10 | 10 | 10 | | |
| Female | | | | | | | | |
| Mean | 1.0 | 1.2 | 0.5 | 0.2 | 1.1 | 0.8 | * | - |
| Std. Err. | 0.2 | 0.1 | 0.1 | 0.1 | 0.2 | 0.2 | | |
| N | 10 | 8 | 10 | 10 | 8 | 9 | | |
| Combined | | | | | | | | |
| Mean | 1.0 | 1.1 | 0.4 | 0.2 | 0.7 | 0.5 | | |
| Std. Err. | 0.1 | 0.1 | 0.1 | 0.1 | 0.1 | 0.1 | | |
| N | 20 | 18 | 20 | 20 | 18 | 19 | | |

| | Monocyte (%) | | | | | | | |
|---|---|---|---|---|---|---|---|---|
| AZT | 0 mg/kg | 0 mg/kg | 50 mg/kg | 500 mg/kg | 50 mg/kg | 500 mg/kg | AZT Dose | Response at |
| Procysteine | 0.0% | 1.0% | 0.0% | 0.0% | 1.0% | 1.0% | -0.0% | -1.0% |
| Male | | | | | | | | |
| Mean | 0.0 | 0.0 | 0.1 | 0.1 | 0.1 | 0.2 | | |
| Std. Err. | 0.0 | 0.0 | 0.0 | 0.0 | 0.1 | 0.0 | | |
| N | 10 | 10 | 10 | 10 | 10 | 10 | | |
| Female | | | | | | | | |
| Mean | 0.1 | 0.1 | 0.1 | 0.1 | 0.1 | 0.1 | | |
| Std. Err. | 0.1 | 0.1 | 0.0 | 0.0 | 0.0 | 0.0 | | |
| N | 10 | 8 | 10 | 10 | 8 | 9 | | |
| Combined | | | | | | | | |
| Mean | 0.1 | 0.0 | 0.1 | 0.1 | 0.1 | 0.1 | - | - |
| Std. Err. | 0.0 | 0.0 | 0.0 | 0.0 | 0.0 | 0.0 | | |
| N | 20 | 18 | 20 | 20 | 18 | 19 | | |

Table 8: Bone Marrow-Miscellaneous Cells

Listing of Mean and Standard Error; * Indicates Significance at Alpha = 0.1

- Indicates Not Significant, Blank Indicates No Statistical Test Performed

Rubriblast (%)

| AZT | 0 mg/kg | 0 mg/kg | 50 mg/kg | 500 mg/kg | 50 mg/kg | 500 mg/kg | AZT Dose | Response at |
|---|---|---|---|---|---|---|---|---|
| Procysteine | 0.0% | 1.0% | 0.0% | 0.0% | 1.0% | 1.0% | -0.0% | -1.0% |
| **Male** | | | | | | | | |
| Mean | 0.2 | 0.0 | 0.3 | 0.4 | 0.4 | 0.4 | * | * |
| Std. Err. | 0.0 | 0.0 | 0.1 | 0.1 | 0.1 | 0.1 | | |
| N | 10 | 10 | 10 | 10 | 10 | 10 | | |
| **Female** | | | | | | | | |
| Mean | 0.1 | 0.3 | 0.1 | 0.1 | 0.3 | 0.3 | - | - |
| Std. Err. | 0.0 | 0.1 | 0.0 | 0.0 | 0.1 | 0.1 | | |
| N | 10 | 8 | 10 | 10 | 8 | 9 | | |
| **Combined** | | | | | | | | |
| Mean | 0.1 | 0.1 | 0.2 | 0.3 | 0.3 | 0.3 | | |
| Std. Err. | 0.0 | 0.1 | 0.0 | 0.0 | 0.0 | 0.1 | | |
| N | 20 | 18 | 20 | 20 | 18 | 19 | | |

Mast Cell (%)

| AZT | 0 mg/kg | 0 mg/kg | 50 mg/kg | 500 mg/kg | 50 mg/kg | 500 mg/kg | AZT Dose | Response at |
|---|---|---|---|---|---|---|---|---|
| Procysteine | 0.0% | 1.0% | 0.0% | 0.0% | 1.0% | 1.0% | -0.0% | -1.0% |
| **Male** | | | | | | | | |
| Mean | 0.0 | 0.0 | 0.0 | 0.0 | 0.0 | 0.0 | | |
| Std. Err. | 0.0 | 0.0 | 0.0 | 0.0 | 0.0 | 0.0 | | |
| N | 10 | 10 | 10 | 10 | 10 | 10 | | |
| **Female** | | | | | | | | |
| Mean | 0.0 | 0.1 | 0.0 | 0.0 | 0.0 | 0.0 | | |
| Std. Err. | 0.0 | 0.0 | 0.0 | 0.0 | 0.0 | 0.0 | | |
| N | 10 | 8 | 10 | 10 | 8 | 9 | | |
| **Combined** | | | | | | | | |
| Mean | 0.0 | 0.0 | 0.0 | 0.0 | 0.0 | 0.0 | - | * |
| Std. Err. | 0.0 | 0.0 | 0.0 | 0.0 | 0.0 | 0.0 | | |
| N | 20 | 18 | 20 | 20 | 18 | 19 | | |

Table 8: Bone Marrow-Miscellaneous Cells

Listing of Mean and Standard Error; * Indicates Significance at Alpha = 0.01

- Indicates Not Significant, Blank Indicates No Statistical Test Performed

Reticulum Cell (%)

| AZT | 0 mg/kg | 0 mg/kg | 50 mg/kg | 500 mg/kg | 50 mg/kg | 500 mg/kg | AZT Dose | Response at |
|---|---|---|---|---|---|---|---|---|
| Procysteine | 0.0% | 1.0% | 0.0% | 0.0% | 1.0% | 1.0% | -0.0% | -1.0% |
| **Male** | | | | | | | | |
| Mean | 0.1 | 0.2 | 0.5 | 0.4 | 0.6 | 0.5 | | |
| Std. Err. | 0.1 | 0.1 | 0.1 | 0.1 | 0.1 | 0.1 | | |
| N | 10 | 10 | 10 | 10 | 10 | 10 | | |

| | | | | | | | | |
|---|---|---|---|---|---|---|---|---|
| **Female** | | | | | | | | |
| Mean | 0.4 | 0.2 | 0.5 | 0.8 | 0.3 | 0.1 | | |
| Std. Err. | 0.2 | 0.1 | 0.1 | 0.1 | 0.1 | 0.0 | | |
| N | 10 | 8 | 10 | 10 | 8 | 9 | | |
| **Combined** | | | | | | | | |
| Mean | 0.3 | 0.2 | 0.5 | 0.6 | 0.4 | 0.3 | * | - |
| Std. Err. | 0.1 | 0.1 | 0.1 | 0.1 | 0.1 | 0.1 | | |
| N | 20 | 18 | 20 | 20 | 18 | 19 | | |

Table 7: Bone Marrow-Myeloid: Erythroid Ratio

Listing of Mean and Standard Error; * Indicates Significance at Alpha = 0.01

- Indicates Not Significant, Blank Indicates No Statistical Test Performed

Myeloid: Erythroid Ratio

| | 0 | 0 | 50 | 500 | 50 | 500 | AZT | Response |
|---|---|---|---|---|---|---|---|---|
| AZT | mg/kg | mg/kg | mg/kg | mg/kg | mg/kg | mg/kg | Dose | at |
| Procysteine | 0.0% | 1.0% | 0.0% | 0.0% | 1.0% | 1.0% | -0.0% | -1.0% |
| **Male** | | | | | | | | |
| Mean | 1.56 | 1.59 | 2.09 | 2.01 | 1.74 | 1.77 | * | * |
| Std. Err. | 0.02 | 0.02 | 0.02 | 0.02 | 0.01 | 0.02 | | |
| N | 10 | 10 | 10 | 10 | 10 | 10 | | |
| **Female** | | | | | | | | |
| Mean | 1.58 | 1.58 | 2.01 | 2.10 | 1.59 | 1.58 | * | - |
| Std. Err. | 0.02 | 0.02 | 0.02 | 0.02 | 0.02 | 0.02 | | |
| N | 10 | 8 | 10 | 10 | 8 | 9 | | |
| **Combined** | | | | | | | | |
| Mean | 1.57 | 1.59 | 2.05 | 2.06 | 1.67 | 1.68 | | |
| Std. Err. | 0.01 | 0.01 | 0.02 | 0.02 | 0.02 | 0.03 | | |
| N | 20 | 18 | 20 | 20 | 18 | 19 | | |

Table 8: Bone Marrow Cell Before Plating

Listing of Mean and Standard Error; * Indicates Significance at Alpha = 0.01

- Indicates Not Significant, Blank Indicates No Statistical Test Performed

Nucleated Cells/Femur (X 10**6)

| | 0 | 0 | 50 | 500 | 50 | 500 | AZT | Response |
|---|---|---|---|---|---|---|---|---|
| AZT | mg/kg | mg/kg | mg/kg | mg/kg | mg/kg | mg/kg | Dose | at |
| Procysteine | 0.0% | 1.0% | 0.0% | 0.0% | 1.0% | 1.0% | -0.0% | -1.0% |
| **Male** | | | | | | | | |
| Mean | 11.8 | 12.8 | 11.7 | 10.5 | 12.6 | 10.0 | | |
| Std. Err. | 0.6 | 0.4 | 0.4 | 0.4 | 0.5 | 0.4 | | |
| N | 10 | 10 | 10 | 10 | 10 | 10 | | |

| | | | | | | | | |
|---|---|---|---|---|---|---|---|---|
| Female | | | | | | | | |
| Mean | 11.4 | 12.0 | 9.9 | 8.8 | 10.4 | 9.0 | | |
| Std. Err. | 0.5 | 0.6 | 0.5 | 0.4 | 0.6 | 0.4 | | |
| N | 10 | 9 | 10 | 10 | 9 | 10 | | |
| Combined | | | | | | | | |
| Mean | 11.6 | 12.4 | 10.8 | 9.6 | 11.5 | 9.5 | * | * |
| Std. Err. | 0.4 | 0.3 | 0.4 | 0.3 | 0.5 | 0.3 | | |
| N | 20 | 19 | 20 | 20 | 19 | 20 | | |

Table 9: Colony Forming Units
Listing of Mean and Standard Error; * Indicates Significance at Alpha = 0.01
- Indicates Not Significant, Blank Indicates No Statistical Test Performed
Myeloid (No. of Colonies/10**4 Cells Plates)

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT Dose -0.0% | Response at -1.0% |
|---|---|---|---|---|---|---|---|---|
| Male | | | | | | | | |
| Mean | 35.6 | 33.9 | 32.6 | 40.7 | 37.6 | 42.2 | | |
| Std. Err. | 1.8 | 1.4 | 2.5 | 2.6 | 1.7 | 3.3 | | |
| N | 10 | 10 | 10 | 10 | 10 | 10 | | |
| Female | | | | | | | | |
| Mean | 34.7 | 32.2 | 35.5 | 46.0 | 31.7 | 43.4 | | |
| Std. Err. | 1.7 | 1.6 | 1.8 | 3.1 | 2.3 | 2.4 | | |
| N | 10 | 9 | 10 | 10 | 9 | 10 | | |
| Combined | | | | | | | | |
| Mean | 35.1 | 33.1 | 34.0 | 43.4 | 34.8 | 42.8 | * | * |
| Std. Err. | 1.2 | 1.0 | 1.5 | 2.1 | 1.5 | 2.0 | | |
| N | 20 | 19 | 20 | 20 | 19 | 20 | | |

Table 9: Colony Forming Units
Listing of Mean and Standard Error; * Indicates Significance at Alpha = 0.01
- Indicates Not Significant, Blank Indicates No Statistical Test Performed
Total (No. of Colonies/10**4 Cells Plated)

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT Dose -0.0% | Response at -1.0% |
|---|---|---|---|---|---|---|---|---|
| Male | | | | | | | | |
| Mean | 39.5 | 37.1 | 36.2 | 44.9 | 41.9 | 46.5 | | |
| Std. Err. | 1.9 | 1.4 | 2.5 | 2.8 | 1.7 | 3.3 | | |
| N | 10 | 10 | 10 | 10 | 10 | 10 | | |

| | | | | | | | | |
|---|---|---|---|---|---|---|---|---|
| Female | | | | | | | | |
| Mean | 38.3 | 35.5 | 39.3 | 50.3 | 35.2 | 47.3 | | |
| Std. Err. | 1.7 | 1.6 | 1.8 | 3.4 | 2.2 | 2.5 | | |
| N | 10 | 9 | 10 | 10 | 9 | 10 | | |
| Combined | | | | | | | | |
| Mean | 38.9 | 36.3 | 37.7 | 47.6 | 38.7 | 46.9 | * | * |
| Std. Err. | 1.2 | 1.0 | 1.5 | 2.2 | 1.6 | 2.0 | | |
| N | 20 | 19 | 20 | 20 | 19 | 20 | | |

## Results and Analysis

Terminal blood was evaluated for hematological effects, and bone marrow smears and aspirates were counted and $10^4$ nucleated cells of the aspirate were cultured 14 days to assess hematopoietic progenitor cells. Anemia, evidenced by significant ($p<0.01$) decreases in total hemoglobin concentration, hematocrit and erythrocyte counts, and increases in mean corpuscular volume, mean corpuscular hemoglobin and the incidence of polychromasia, anisocytosis, macrocytes and Howell-Jolly bodies, was similarly observed across both sexes for AZT and AZT/Procysteine®-treated groups.

Bone marrow aspirates exhibited a significant reduction in nucleated cells for AZT and high-dose AZT/Procysteine® -treated groups compared to controls. No group differences were noted in burst forming units-erythroid for marrow cultures, however, a significant induction was observed in total colonies and colony forming units-granulocyte/macrophage for AZT and AZT/Procysteine® groups.

Bone marrow smears of AZT-treated mice had a reduced total count of erythroid series cells due to a marked reduction of metarubricytes (nonproliferating) and a mild reduction of rubriblasts. AZT/Procysteine®-treated mice had a significantly less severe decrease in erythroid counts than did the AZT-only treated animals, with the rubriblast, rubricyte and metarubricyte (female) counts being similar to control values.

AZT significantly increased the proliferating compartment of the myeloid series, with a reduction in the nonproliferating compartment. The AZT/Procysteine®-treated mice had a less severe increase in the proliferating myeloid compartment compared to the AZT-only treated mice. The myeloid:erythroid ratios for AZT groups were significantly increased whereas those for AZT/Procysteine® groups were at control level.

In conclusion, these results indicate that the coadministration of Procysteine® with AZT should reduce at least some indices of AZT-induced hematotoxicity.

## EXAMPLE NO. 2

The purpose of this study was to examine the *in vitro* efficacy of Procysteine® (L-2-oxothiazolidine-4-carboxylate) alone and in combination with other approved antivirals (i.e., AZT, ddI) to inhibit human immunodeficiency virus (HIV) infection.

Three cell culture systems were used in this study: H-9 cells; MT-2 cells; and normal human peripheral blood mononuclear cells (PBMC). H-9 cells were obtained from Advanced Biotechnology Inc. (ABI). MT-2 cells were obtained from Dr. J. Armstrong of the University of Pittsburgh. MT-2 cells are HTLV-I transformed human T lymphocytes and continuously produce HTLV-I virions. PBMC were obtained from healthy donors, purified by Ficol-Hypaque, and stimulated with PHA mitogen before being used in the efficacy studies. The initial stock of HIV-1 (strain $III_B$) used in this study was obtained from ABI and the working stocks were produced. The drugs tested in this study were either obtained from Clintec Nutrition Company, Deerfield, Illinois (AZT and Procysteine®) or from Sigma (ddI).

## Materials

The following doses were tested to determine the median effective dose ($ED_{50}$) of different drugs: for AZT: 0.0025, 0.01, 0.04, 0.16, and 0.64 μM; for ddI: 0.25, 1, 4, 16, and 64 μM; and for Procysteine®: 0.023, 0.23, 0.93, 3.75, 15, and 60 mM. The effect of Procysteine® in combination with other approved antivirals (i.e., AZT, ddI) to inhibit HIV infection was evaluated under two different treatment of either 25 mM or 50 mM of Procysteine® with a matrix of full strength, 3/4, 1/2 and 1/4 of the $ED_{50}$ dose of AZT of ddI (0.032 μM of AZT was used as the single treatment $ED_{50}$ doses of AZT in PBMC, Table 7).

At the completion of this evaluation, a toxicity study conducted concomitantly revealed that the median lethal doses ($LD_{50}$) of Procysteine® for the cell systems studied in this protocol were in the range of 5-30 mM. Therefore, the combination treatments were re-evaluated with a matrix of AZT (at 0.0025, 0.01, 0.04, 0.16, and 0.64 μM) or ddI (at 0.06, 0.25, 1, 4, and 16 μM) combined with Procysteine® (at 0.1, 1, 5, and 10 mM).

Procedures:

The antiviral efficacies of Procysteine®, ddI and AZT on HIV were examined individually in all three cell cultures (H-9, MT-2, and PBMC). Cells were collected, put into tissue culture plates and incubated with different concentrations of drugs in RPMI 1640 tissue culture medium for 20 minutes prior to HIV challenge. Infection was carried out by inoculating 20-100 HIV infectious units (IU)/well inoculum without removing the drugs. The untreated cells exposed to HIV challenge served as positive controls. The normal untreated uninfected cells served as negative controls.

Both the MTT assay and HIV p24 ELISA were used for evaluation of drug efficacies with MT-2 cells. The MTT assay is based upon the metabolic reduction of the MTT tetrazolium bromide dye in live cells. The OD readings (at 540 nm) obtained from the MTT assay are inversely correlated to the levels of cell mortality due to HIV infection or the cytotoxic effect of drugs. For a drug treated sample, a higher MTT OD reading reflects that the drug is more effective in protecting cells against acute HIV infection. The HIV p24 antigen assay measures the level of HIV antigen present in the sample (i.e., culture supernatant). A higher comparative OD reading (at 450 nm) indicates that more HIV is present in the sample. Therefore, higher HIV p24 readings in treated samples reflect lower effectiveness of the drug in protecting cells against HIV acute infection.

When evaluating the efficacies of drugs in H-9 cells and PBMC systems, only HIV p24 assays were used. To calculate the $ED_{50}$ values, a second order linear regression was applied on the logarithm of the tested doses of different drugs against the OD readings obtained from either MTT assays or HIV p24 assays.

In the protocol, combination treatment was originally designed to be evaluated with a matrix of full strength, 3/4, 1/2, and 1/4 of the single treatment $ED_{50}$ doses of Procysteine®, AZT, or ddI after these values were obtained. However, since every single treatment $ED_{50}$ value for Procysteine® was much higher than the median lethal dose ($LD_{50}$), a deviation was made in an attempt to avoid cytotoxicity of Procysteine® to the test cells. The combination treatment was performed by treatment of cells with either 25 mM or 50 mM of Procysteine® mixed with full strength, 3/4, 1/2, and 1/4 of the single treatment $ED_{50}$ dose of AZT or ddI. These doses of Procysteine® were chosen based upon what was, at the time, thought to be the maximal tolerant dose of Procysteine® (i.e., 50 mM).

Cells were collected, put into tissue culture plates and incubated with different combinations of drugs for 20 minutes prior to HIV infection in the combined treatment studies. Infection was carried out by inoculating 20-100 HIV IU/well inoculum without removing the drugs. The inocula were the same as the ones used in the single drug treatment to obtain the $ED_{50}$ values. Both MTT assays and HIV p24 antigen assays were used in evaluation of drug effects in MT-2 cells but only HIV p24 antigen assays were used for H-9 cells or PBMC.

It was found that the HIV p24 antigen assay used in the MT-2 cell systems provided more consistent results. Therefore, only the results from the HIV p24 assays from all three cell systems were used to analyze the role of Procysteine® in combined treatment.

At the completion of the assessment on combination treatment of Procysteine® with AZT or ddI, a separate toxicity study revealed that the Procysteine® doses tested (i.e., 25 and 50 mM) were still toxic to all three cell systems. The median lethal doses ($LD_{50}$) of single treatment of Procysteine® were 5 mM with PBMC, 21 mM with H-9 cells, and 31 mM with MT-2 cells. The $LD_{50}$ of Procysteine® when it was evaluated in combination with 1 μM ddI were similar to the $LD_{50}$ of single treatment in all three cells. Therefore, both 25 mM or 50 mM of Procysteine® could have caused significant cell mortality in the test cell systems. This toxic effect would reduce the levels of HIV production due to the loss of host cells and hence generate unreal drug efficacies. To avoid the misinterpretation of drug antiviral efficacy due to toxicity, an alternative design using lower concentrations of Procysteine® in the combination treatment was performed. The treatment was studied by a combination treatment matrix of AZT (at 0.0025, 0.01, 0.04, 0.16, and 0.64 μM) or ddI (at 0.06, 0.25, 1, 4, and 16 μM) with Procysteine® (at 0.1, 1, 5, and 10 mM) in all three cell systems. The HIV p24 assays were performed for analysis.

Results and Discussion:

$ED_{50}$ of Procysteine®, ddI, and AZT

The OD readings obtained from single treatment with AZT, ddI, or Procysteine® in H-9, MT-2, and PBMC are shown in Table 10. Both an MTT assay and an HIV p24 antigen assay were used to evaluate samples collected from H-9 cells or PBMC. Table 11 depicts the $ED_{50}$ values of different drugs. The $ED_{50}$ values were calculated by performing linear regressions on the logarithms of tested doses against OD readings. The efficacies of AZT or ddI single treatment were also evaluated in the subsequent combination treatment studies following the acknowledgement of the $LD_{50}$ from the toxicity study performed and are shown in Table 12. The single treatment $ED_{50}$ values of AZT or ddI calculated from this re-evaluation are summarized in Table 13.

The $ED_{50}$ values observed in MT-2 cells, challenged with 100 IU/well HIV and measured by MTT assay, were 0.055 μM for AZT, 2.778 μM for ddI, and 2.3 x $10^{13}$ mM for Procysteine®. The $ED_{50}$ values observed in MT-2 cells challenged with 20 IU/well HIV and measured by HIV p24 assay were 0.072-0.081 μM for AZT, 4.069-12.24 μM for ddI, and 1613 mM for Procysteine® (Tables 11 & 13).

The $ED_{50}$ values observed in H-9 cells, challenged with 80 IU/well HIV, were 0.105-0.616 μM for AZT, 2.10-2.424 μM for ddI, and 653 mM for Procysteine®. The $ED_{50}$ values observed in PBMC, challenged with 50 IU/well HIV, were 0.021-0.050 μM for AZT,, 0.241-1.524 μM for ddI, and 378 mM for Procysteine® (Tables 11 & 13). These results indicate that AZT was most effective in PBMC and least effective in H-9 cells; ddI worked equally well in these three cell systems. Procysteine® was most effective in PBMC and least effective in MT-2 cells, however, all of the calculated $ED_{50}$ values for Procysteine® were much higher than the $LD_{50}$ in the cognate cell systems.

## Synergistic Effect of Procysteine to ddi and AZT

Since the single treatment $ED_{50}$ values of Procysteine® were much higher than the $LD_{50}$ of Procysteine®, the combination treatments were done in doses of Procysteine® at or below the $LD_{50}$ values. The study was first performed by using combination treatment of 25 mM or 50 mM of Procysteine® with AZT or ddI (Tables 14-16) and was carried out again with a lower Procysteine® level (0.1 mM, 1 mM, 5 mM, and 10 mM, Tables 18-20). The $ED_{50}$ values of AZT or ddI at a specific Procysteine® dose were calculated following the same formula as used for the single treatment $ED_{50}$ calculation. The $ED_{50}$ values of AZT or ddI in combination treatment with Procysteine® at varying doses are summarized in Table 17 and Table 21.

To analyze the effect of Procysteine® in combination treatment, the combination index (CI) for mutually exclusive drugs suggested by T. Chou and P. Talalay was applied. Per Chou and Talalay, CI is the sum of the ratios between the single treatment median effective dose and the combined treatment median effective dose for each drug (two or more drugs) tested in the combined treatment. When two mutually exclusive drugs have a synergistic effect, the $ED_{50}$ values obtained from combination treatment should be lower than $ED_{50}$ values from single treatment.

Therefore, if the CI is less than 1, it indicates synergism among the tested drugs. Similarly, if the CI is equal to 1, the effects are additive, while a CI value greater than 1 is indicative of an antagonistic effect. $ED_{50}$ values listed in Table 11, 13, 17, or 18 were used in the calculation of the CI and the results are summarized in Table 22. Since in every combination test, the $ED_{50}$ values of Procysteine® were not available (much higher than $LD_{50}$ values), the actual amount of Procysteine® used in the test was equivalent to the $ED_{50}$ for Procysteine in combination treatment.

A significant synergism was observed at low concentrations of Procysteine® with AZT in PBMC. Although the $LD_{50}$ value of Procysteine® with 1 μM of AZT in PBMC was lower than that in MT-2 cells or H-9 cells (8.3 mM versus 36 mM or 30 mM), the CIs in the presence of mM of Procysteine® indicate significant synergistic effects between these low doses of Procysteine® and AZT (CI of 0.01 and 0.18).

The role of Procysteine® in the combination treatment with ddI is inconclusive in all three cell systems. In MT-2 cells, antagonism was observed when 0.1-1 mM Procysteine® was evaluated with ddI combination treatment. However, synergism was observed when 5-10 mM of Procysteine® was evaluated (these doses are lower than the $LD_{50}$ value, 31-32 mM). The drops of CIs at 25 mM and 50 mM of Procysteine® with ddI in MT-2 cells, again, indicate the increased toxic effects of Procysteine® to the cells. Similarly, mixed results of antagonism and synergism were observed in H-9 cells and in PBMC when the combination treatments were evaluated at non-toxic doses of Procysteine®.

## Conclusion

The *in vitro* antiviral efficacy of Procysteine® and its effect in combination with other approved antivirals (i.e., AZT, ddI) to inhibit human immunodeficiency virus (HIV) infection were evaluated. A very significant synergism was observed in the combination treatment of Procysteine® with AZT in inhibiting acute HIV infection in PHA stimulated normal human peripheral leukocytes (PBMC).

TABLE 10

OD readings from single treatment of Procysteine®, ddI and AZT for $ED_{50}$ calculation.[1]

| Drug | Conc. | Cell lines | | | |
|---|---|---|---|---|---|
| | | MT-2[2] | MT-2[3] | H-9[3] | PBMC[3] |
| AZT | 0.0025 µM | 0.474 | 2.703 | 4.133 | 3.405 |
| | 0.01 µM | 0.641 | 2.353 | 4.133 | 2.978 |
| | 0.04 µM | 0.678 | 2.280 | 4.133 | 0.155 |
| | 0.16 µM | 0.688 | 1.490 | 3.815 | 0.163 |
| | 0.64 µM | 0.946 | 1.292 | 0.358 | 0.204 |
| ddI | 0.25 µM | 0.551 | 3.351 | 3.984 | 2.730 |
| | 1 µM | 0.603 | 3.025 | 1.542 | 2.658 |
| | 4 µM | 0.561 | 0.710 | 0.772 | 0.672 |
| | 16 µM | 0.880 | 1.167 | 0.716 | 0.212 |
| | 64 µM | 1.135 | 1.131 | 0.329 | 0.098 |
| Procysteine | 0.023 mM | 0.392 | 3.269 | 4.133 | 3.703 |
| | 0.23 mM | 0.265 | 3.712 | 4.133 | 3.057 |
| | 0.93 mM | 0.283 | 3.762 | 4.133 | 2.951 |
| | 3.75 mM | 0.270 | 3.649 | 4.133 | 3.640 |
| | 15 mM | 0.308 | 2.825 | 4.099 | 3.284 |
| | 60 mM | 0.350 | 1.675 | 0.767 | 1.141 |
| V.C. | | 0.403 | 3.696 | 3.505 | 3.616 |
| C.C. | | 1.013 | 0.042 | 0.020 | 0.005 |

1. The challenge titer of HIV used in the studies were: 100 IU/well for MT-2 cells using the MTT assay and 20 IU/well for the HIV p24 assay, 80 IU/well for H-9 cells, and 50 IU/well for PBMC.
2. The MTT tetrazolium bromide dye (Thiazolyl blue; 3-[4,5-Dimethylthiazol-2-yl]-2,5-diphenyltetrazolium bromide) uptake assay was used. The study was performed on Day 6 cultures. OD readings at 540 nm from different groups shown in the Table.
3. The HIV p24 antigen capture kits from Coulter were used. OD readings at 450 nm from different groups are shown in the Table.

TABLE 11

Calculated single treatment $ED_{50}$ of AZT, ddI and Procysteine[1].

| Drug | MT-2 cells[2] | | H-9 cells | PBMC |
|---|---|---|---|---|
| | MTT assay | p24 assay | | |
| AZT | 0.055 μM | 0.072 μM | 0.616 μM | 0.021 μM |
| ddI | 2.778 μM | 4.069 μM | 2.424 μM | 1.524 μM |
| Procysteine | $2.3 \times 10^{13}$ mM | 1613 mM | 653 mM | 378 mM |

1. $ED_{50}$ values were calculated by the second order linear regression of the logarithm of tested doses and the OD readings (Table 10) by MTT assay or by HIV p24 assay.

2. Both MTT assay (challenged with 100 IU/well HIV) and HIV p24 assay (challenged with 20 IU/well HIV) were used for the evaluation of MT-2 cells. The HIV p24 assay was used to evaluate H-9 cells (challenged with 80 IU/well HIV) or PBMC (challenged with 50 IU/well HIV).

TABLE 12

OD readings from single treatment of ddI and AZT for $ED_{50}$ calculation.[1]

| Drug | Conc. | Cell lines | | |
|---|---|---|---|---|
| | | MT-2[2] | H-9[2] | PBMC[2] |
| AZT | 0.0025 μM | 3.354 | 3.633 | 3.936 |
| | 0.01 μM | 4.078 | 3.307 | 2.903 |
| | 0.04 μM | 3.104 | 2.227 | 2.48 |
| | 0.16 μM | 0.768 | 1.591 | 1.698 |
| | 0.64 μM | 0.841 | 1.126 | 0.211 |
| ddI | 0.06 μM | 3.861 | 3.688 | 3.778 |
| | 0.25 μM | 3.888 | 3.558 | 1.76 |
| | 1 μM | 3.231 | 2.572 | 0.203 |
| | 4 μM | 3.901 | 1.005 | 0.093 |
| | 16 μM | 0.744 | 0.904 | 0.215 |
| V.C. | | 4.013 | 3.81 | 4.193 |
| C.C. | | 0 | 0.008 | 0.016 |

1. Results obtained from the re-evaluation of drug efficacies following the acknowledgement of $LD_{50}$ values are shown in this Table.
2. These challenge titer of HIV used in the studies were: 20 IU/well for MT-2 cells, 80 IU/well for H-9 cells, and 50 IU/well for PBMC. The HIV p24 antigen capture kits from Coulter were used to evaluate the samples.

TABLE 13

Calculated single treatment $ED_{50}$ of AZT and ddI.[1]

| Drug | MT-2 cells | H-9 cells | PBMC |
|---|---|---|---|
| AZT | 0.081 μM | 0.105 μM | 0.050 μM |
| ddI | 12.24 μM | 2.10 μM | 0.241 μM |

1. $ED_{50}$ values were calculated by the second order linear regression of the logarithm of tested doses and the OD readings (Table 12). The OD readings shown in Table 12 were obtained from the re-evaluation of drug efficacies following the acknowledgement of $LD_{50}$ values.

TABLE 14

$OD_{450}$ readings from combination treatment of AZT or ddI with 25 or 50 mM Procysteine® in MT-2 cells.

| Drug | Conc. | Procysteine® conc. | |
|---|---|---|---|
| | | 50 mM | 25 mM |
| AZT | 0.070 μM | 0.536 | 0.462 |
| | 0.053 μM | 0.554 | 0.790 |
| | 0.035 μM | 1.010 | 1.237 |
| | 0.018 μM | 0.629 | 1.838 |
| ddI | 4.070 μM | 0.513 | 0.951 |
| | 3.052 μM | 0.612 | 0.650 |
| | 2.040 μM | 0.722 | 1.587 |
| | 1.018 μM | 1.050 | 2.326 |
| V.C. | | 2.472 | |
| C.C. | | 0.0 | |

1. The Coulter HIV p24 antigen capture assay kit was used. OD readings at 450 nm from different groups are shown in the Table.
2. 20 IU/well inoculum was used to challenge MT-2 cells with different drug combination treatment.

$ED_{50}$ value of 1617 mM in H-9 cells, thus, 25 and 50 mM were tested in this study.

TABLE 15

$OD_{450}$ readings from combination treatment of AZT or ddI with 25 or 50 mM Procysteine® in H-9 cells.

| Drug | Conc. | Procysteine® conc. | |
|---|---|---|---|
| | | 50 mM | 25 mM |
| AZT | 0.62 μM | 0.502 | 0.553 |
| | 0.47 μM | 0.477 | 0.916 |
| | 0.31 μM | 0.662 | 0.881 |
| | 0.16 μM | 0.656 | 1.817 |
| ddI | 2.43 μM | 0.417 | 0.543 |
| | 1.82 μM | 0.436 | 1.118 |
| | 1.22 μM | 0.460 | 1.443 |
| | 0.61 μM | 1.589 | 2.448 |
| V.C. | | 3.427 | |
| C.C. | | 0.0 | |

1. The Coulter HIV p24 antigen capture assay was used. OD readings at 450 nm from different groups are shown in the Table.
2. 80 IU/well inoculum was used to challenge H-9 cells with different drug combination treatment. $ED_{50}$ derived from single drug testing and being tested in this study were: AZT: 0.62 μM, ddI: 2.43 μM. Procysteine® has a $ED_{50}$ value in H-9 cells of greater than 100 mM (651 mM), thus, 25 and 50 mM were tested in this study.

TABLE 16

$OD_{450}$ readings from combination treatment of AZT or ddI with 25 or 50 mM Procysteine® in PBMC cells.

| Drug | Conc. | Procysteine® conc. | |
|---|---|---|---|
| | | 50 mM | 25 mM |
| AZT | 0.032 μM | 0.174 | 0.275 |
| | 0.024 μM | 0.322 | 0.514 |
| | 0.016 μM | 0.284 | 0.425 |
| | 0.008 μM | 1.686 | 0.367 |
| ddI | 1.52 μM | 0.258 | 0.288 |
| | 1.14 μM | 0.293 | 0.909 |
| | 0.76 μM | 0.245 | 0.954 |
| | 0.38 μM | 0.417 | 1.511 |
| V.C. | | 2.583 | |
| C.C. | | 0.048 | |

1. The Coulter HIV p24 antigen capture assay was used. The OD readings at 450 nm from different groups are shown in the Table.
2. 50 IU/well inoculum was used to challenge peripheral blood mononuclear cells (PBMC) with different drug combination treatment. $ED_{50}$ derived from single drug testing and being tested in this study were: AZT: 0.03 μM, ddI: 1.52 μM. Procysteine® has a $ED_{50}$ value of 379 mM in PBMC, thus, 25 and 50 mM were tested in this study.

TABLE 17

Calculated combination treatment $ED_{50}$ of Procysteine® or ddI.[1]

| Drug | Procysteine® Conc. | MT-2 cells | H-9 cells | PBMC |
|---|---|---|---|---|
| AZT | 25 mM | 0.034 μM | 0.160 μM | $3.2 \times 10^{-30}$ μM |
| | 50 mM | 0.024 μM | 0.004 μM | 0.009 μM |
| ddI | 25 mM | 2.550 μM | 1.053 μM | 0.505 μM |
| | 50 mM | 0.598 μM | 0.428 μM | $5.7 \times 10^{-5}$ μM |

1. $ED_{50}$ values were calculated by the second order linear regression of the logarithm of tested doses and the OD readings (Table 14, 15, or 16) by HIV p24 assays.

TABLE 18

$OD_{450}$ readings from combined treatment of Procysteine® with AZT or ddI in MT-2 cells.[1]

| Drug | Conc. | Procysteine® Concentration | | | |
|---|---|---|---|---|---|
| | | 0.1 mM | 1 mM | 5 mM | 10 mM |
| AZT | 0.0025 μM | 3.411 | 4.150 | 3.712 | 3.735 |
| | 0.01 μM | 3.744 | 4.055 | 3.870 | 4.15 |
| | 0.04 μM | 3.241 | 2.21 | 2.221 | 2.191 |
| | 0.16 μM | 1.022 | 0.675 | 1.32 | 0.957 |
| | 0.64 μM | 0.676 | 1.126 | 0.935 | 0.962 |
| | V.C. | 4.013 | | | |
| | C.C. | 0 | | | |
| ddI | 0.06 μM | 4.012 | 4.038 | 4.018 | 3.829 |
| | 0.25 μM | 3.88 | 4.15 | 4.15 | 4.121 |
| | 1 μM | 4.091 | 3.991 | 2.874 | 1.992 |
| | 4 μM | 3.042 | 3.721 | 3.261 | 1.587 |
| | 16 μM | 1.347 | 1.256 | 0.915 | 0.957 |
| | V.C. | 4.013 | | | |
| | C.C. | 0 | | | |

1. Results obtained from the experiment performed are shown in the Table. 20 IU/well inoculum was used to challenge MT-2 cells with different drug combination treatment. The Coulter HIV p24 antigen capture assay was used. OD readings at 450 nm from different groups are shown in the Table.

TABLE 19

$OD_{450}$ readings from combined treatment of Procysteine® with AZT or ddI in H-9 cells.[1]

| Drug | Conc. | Procysteine® Concentration | | | |
|---|---|---|---|---|---|
| | | 0.1 mM | 1 mM | 5 mM | 10 mM |
| AZT | 0.0025 µM | 3.604 | 3.733 | 3.755 | 3.707 |
| | 0.01 µM | 3.528 | 2.803 | 3.605 | 3.34 |
| | 0.04 µM | 3.091 | 2.987 | 2.61 | 2.4 |
| | 0.16 µM | 1.901 | 1.555 | 1.17 | 1.604 |
| | 0.64 µM | 1.43 | 1.207 | 1.354 | 1.127 |
| | V.C. | 3.81 | | | |
| | C.C. | 0.008 | | | |
| ddI | 0.06 µM | 3.841 | 3.788 | 3.885 | 3.831 |
| | 0.25 µM | 3.775 | 3.561 | 3.561 | 3.819 |
| | 1 µM | 2.884 | 2.533 | 2.003 | 1.489 |
| | 4 µM | 1.113 | 1.418 | 1.203 | 1.106 |
| | 16 µM | 1.169 | 1.306 | 1.092 | 1.102 |
| | V.C. | 3.81 | | | |
| | C.C. | 0.008 | | | |

1. Results obtained from experiment performed are shown in the Table. 80 IU/well inoculum was used to challenge H-9 cells with different drug combination treatment. The Coulter HIV p24 antigen capture assay was used. OD readings at 450 nm from different groups are shown in the Table.

TABLE 20

$OD_{450}$ readings from combined treatment of Procysteine® with AZT or ddI in PBMC.[1]

| Drug | Conc. | Procysteine® Concentration | | | |
|---|---|---|---|---|---|
| | | 0.1 mM | 1 mM | 5 mM | 10 mM |
| AZT | 0.0025 μM | 1.588 | 3.241 | 3.897 | 3.54 |
| | 0.01 μM | 1.398 | 0.67 | 1.623 | 2.964 |
| | 0.04 μM | 0.653 | 0.991 | 0.665 | 1.505 |
| | 0.16 μM | 0.583 | 0.258 | 0.431 | 0.148 |
| | 0.64 μM | 0.132 | 0.016 | 0.014 | 0.108 |
| | V.C. | 4.193 | | | |
| | C.C. | 0.016 | | | |
| ddI | 0.06 μM | 2.05 | 4.193 | 4.101 | 3.983 |
| | 0.25 μM | 3.348 | 3.616 | 2.012 | 1.747 |
| | 1 μM | 0.216 | 0.239 | 0.576 | 0.634 |
| | 4 μM | 0.227 | 0.241 | 0.233 | 0.259 |
| | 16 μM | 0.196 | 0.228 | 0.225 | 0.24 |
| | V.C. | 4.193 | | | |
| | C.C. | 0.016 | | | |

1. Results obtained from experiment performed are shown in the Table. 50 IU/well inoculum was used to challenge PBMC with different drug combination treatment. The Coulter HIV p24 antigen capture assay was used. OD readings at 450 nm from different groups are shown in the Table.

TABLE 21

Calculated combination treatment $ED_{50}$ of Procysteine® with AZT or ddI.[1]

| Drug | Procysteine® Conc. | MT-2 cells | H-9 cells | PBMC |
|---|---|---|---|---|
| AZT | 0.1 mM | 0.080 μM | 0.257 μM | 0.0004 μM |
| | 1 mM | 0.076 μM | 0.134 μM | 0.005 μM |
| | 5 mM | 0.080 μM | 0.124 μM | 0.012 μM |
| | 10 mM | 0.075 μM | 0.115 μM | 0.021 μM |

| | | | | |
|---|---|---|---|---|
| ddI | 0.1 mM | 17.55 μM | 3.07 μM | 0.158 μM |
| | 1 mM | 27.57 μM | 3.30 μM | 0.605 μM |
| | 5 mM | 7.64 μM | 2.15 μM | 0.370 μM |
| | 10 mM | 2.27 μM | 1.84 μM | 0.319 μM |

1. $ED_{50}$ values were calculated by the second order linear regression of the logarithm of tested doses and the OD readings (Table 18, 19, or 20) by HIV p24 assays.

TABLE 22

Combination Index (CI) of Procysteine® in combination treatment with AZT or ddI.[1]

| Drug | Procysteine® Conc. | MT-2 cells | H-9 cells | PBMC |
|---|---|---|---|---|
| AZT | 0.1 mM | 0.99 | 2.45 | 0.01 |
| | 1 mM | 0.94 | 1.28 | 0.10 |
| | 5 mM | 0.99 | 1.19 | 0.25 |
| | 10 mM | 0.93 | 1.11 | 0.47 |
| | 25 mM | 0.49 | 0.30 | 0.07 |
| | 50 mM | 0.36 | 0.08 | 0.56 |
| ddI | 0.1 mM | 1.43 | 1.46 | 0.66 |
| | 1 mM | 2.25 | 1.57 | 2.51 |
| | 5 mM | 0.63 | 1.03 | 1.55 |
| | 10 mM | 0.19 | 0.89 | 1.35 |
| | 25 mM | 0.64 | 0.47 | 0.40 |
| | 50 mM | 0.18 | 0.25 | 0.13 |

1. Combination index (CI) is a term suggested by T. Chou and P. Talalay to quantify the synergism, summation, or antagonism of multiple drugs. The formula to calculate CI for two mutually exclusive drugs is $CI = (D)_1/(ED_{50})_1 + (D)_2/(ED_{50})_2$ where $(D)_x$ is the median effective dose ($ED_{50}$) of different drugs when tested in combination, and $(ED_{50})_x$ is the $ED_{50}$ of

different drugs when tested individually. If CI > 1 antagonism is indicated, if CI = 1 summation is indicated, and if CI < 1 synergism is indicated. .

2. To calculate CI, the $ED_{50}$ values listed in Table 2, 4, 8, or 12 are used. Since in every combination test, the $ED_{50}$ values of Procysteine® was not available (much higher than $LD_{50}$ values), the actual amount of Procysteine® used in the test was applied as (D) for Procysteine®. For example, to calculate the CI of ddI and 1 mM Procysteine® in PBMC, the following formula was used: 0.605 μM/0.241 μM + 1 mM/379 mM = 2.51.

It should be understood that various changes and modifications to the presently preferred embodiments described herein will be apparent to those skilled in the art. Such changes and modifications can be made without departing from the spirit and scope of the present invention and without diminishing its attendant advantages. It is therefore intended that such changes and modifications be covered by the appended claims.

## Claims

1. Use in the manufacture of a pharmaceutical preparation of a composition that stimulates the intracellular synthesis of glutathione for the purpose of potentiating the antiviral effect of an antiretroviral agent administered to a patient.

2. Use according to Claim 1, wherein the preparation includes the antiretroviral agent.

3. Use in the manufacture of a pharmaceutical preparation containing an antiretroviral agent of a composition that stimulates the intracellular synthesis of glutathione for the purpose of prolonging the life expectancy of a patient who is HIV positive, or is suffering from AIDS.

4. Use according to Claim 1, 2 or 3 wherein the composition is parenterally administrable.

5. Use according to Claim 1, 2 or 3 wherein the composition is enterally administrable.

6. Use according to any preceding claim wherein the composition includes L-2-oxothiazolidine-4-carboxylate, or an ester thereof.

7. Use according to any one of Claims 1 to 5 wherein the composition includes a thiazolidine-4-carboxylate analog.

8. Use according to any one of Claims 1 to 5 wherein the composition includes a glutathione ester.

9. Use according to Claim 8 wherein the composition includes an ester chosen from the group consisting of: a methyl ester of glutathione; an ethyl ester of glutathione; short chain acetyl esters of glutathione; and a glutathione isopropyl ester.

10. Use according to any preceding claim wherein the antiretroviral compound is AZT.

11. Use according to any preceding claim wherein the composition includes proteins rich in cysteine, or N-acetyl cysteine.

12. Use according to Claim 11 wherein the proteins rich in cysteine are chosen from whey, egg white, serum albumin, and lactalbumin.